# EUROPÄISCHE PATENTSCHRIFT

(11) **EP 3 883 469 B1**
(45) Veröffentlichungstag und Bekanntmachung des Hinweises auf die Patenterteilung: **24.12.2025**
(21) Anmeldenummer: 20829182.3
(22) Anmeldetag: 30.11.2020
(51) Int. Cl.: A61B 5/097, A61B 5/00

(54) **LUNGENTESTVORRICHTUNG**
LUNG TESTING DEVICE
DISPOSITIF DE TEST PULMONAIRE

(30) Priorität: 02.12.2019 AT 602662019; 16.04.2020 AT 503232020
(43) Veröffentlichungstag der Anmeldung: 29.09.2021
(73) Patentinhaber: 7S Health.Care GmbH, 1190 Wien (AT)
(72) Erfinder: WEGERER, Alfred, 4040 Linz (AT); SINDHUBER, Gerald, 3363 Ulmerfeld (AT)
(74) Vertreter: Burgstaller, Peter
(86) Internationale Anmeldenummer: PCT/AT2020/060425
(87) Internationale Veröffentlichungsnummer: WO 2021/108822

(56) Entgegenhaltungen:
- EP-A1- 0 373 585
- WO-A1-2004/041084
- WO-A1-2019/168479
- WO-A1-2019/222464
- WO-A2-2008/106961
- DE-U1- 202015 003 822
- US-A1- 2016 121 062
- US-A1- 2018 140 252

## Beschreibung

Die im Folgenden offenbarte Erfindung betrifft eine Vorrichtung zur Ermittlung von Messwerten beschreibend die Funktion einer Lunge eines Patienten nach Anspruch 1. Die hier offenbarte Erfindung betrifft im weiteren Sinn eine Vorrichtung zur Ermittlung von Messwerten beschreibend die Funktion eines respiratorischen Systems im Allgemeinen, im Speziellen der oberen Atemwege wie der Nasenhöhle und der Nasennebenhöhle, der Mundhöhle und des Rachens und/oder der unteren Atemwege wie des Kehlkopfes, der Luftröhre, der Bronchien, der Bronchiolen, der Bronchioli terminales, der Bonchioli repiratorii und der Alveolen.

Es ist nach dem Stand der Technik die Impulsoszillometrie bekannt. Die Impulsoszillometrie ist ein Verfahren zur mechanischen Analyse der Atmung (Lungenfunktionsuntersuchung) und dient insbesondere zur Bestimmung des Atemwegswiderstandes. Über den Atemwegswiderstand hinaus erfasst sie auch die Trägheits- und Dehnbarkeitswiderstände von Lunge und Thorax. Die Ergebnisse können zur Diagnose von Atemwegskrankheiten herangezogen werden (siehe auch Eintrag hierzu auf Wikipedia).

Im Rahmen der Offenbarung der Erfindung sei festgehalten, dass ein Druck sowohl ein positives Vorzeichen (auf eine Fläche oder ein Medium gerichtete Flächenkraft) als auch ein negatives Vorzeichen (von einer Fläche oder einem Medium weg gerichtete Flächenkraft) aufweisen kann.

CN108685575 beschreibt eine Vorrichtung zur Bestimmung einer Gaszusammensetzung einer Atemluft unter Ausübung von Druck auf die Lunge. Dieses Dokument erwähnt nicht den Aufbau des Gassensors. CN108685575 offenbart die Verwendung eines Lautsprechers als Pumpvorrichtung. Dieses Dokument offenbart keinen Gasmessraum, welcher Gasmessraum in einem ersten Gasmessraum und einen zweiten, räumlich abgeschlossenen oder abschließbaren Gasmessraum mit zumindest einem Gassensor umfasst. WO2017194906 offenbart eine Vorrichtung zur Bestimmung einer Gaszusammensetzung einer Atemluft unter Ausübung eines alternierenden Widerstandes auf die ausgegebene Atemluft. Dieses Dokument offenbart keinen Gasmessraum, welcher Gasmessraum in einem ersten Gasmessraum und einen zweiten, räumlich abgeschlossenen oder abschließbaren Gasmessraum mit zumindest einem Gassensor umfasst. EP0017162 erwähnt eine in einem Atembeutel eingebrachte Messsonde zur Bestimmung der Gaszusammensetzung der Atemluft. Der Atembeutel ist als kein räumlich abgeschlossener oder abschließbarer Gasmessraum anzusehen,

WO2013098714 beschreibt die Bestimmung der Gaszusammensetzung der Atemluft unter Beeinflussung des in die Vorrichtung eingebrachten Luftstroms mittels Ventile. Dieses Dokument offenbart keinen Gasmessraum, welcher Gasmessraum in einem ersten Gasmessraum und einen zweiten, räumlich abgeschlossenen oder abschließbaren Gasmessraum mit zumindest einem Gassensor umfasst. WO2010067244 erwähnt die Ermittlung der Gaszusammensetzung der Atemluft unter Berücksichtigung einer Elastizität und eines Widerstandes des Atems der Person. Dieses Dokument offenbart keinen Gasmessraum, welcher Gasmessraum in einem ersten Gasmessraum und einen zweiten, räumlich abgeschlossenen oder abschließbaren Gasmessraum mit zumindest einem Gassensor umfasst.

WO2008106961 offenbart eine Vorrichtung zur Bestimmung der Gaszusammensetzung der Atemluft unter Beaufschlagung der Atemluft oder der Lunge mit einem durch eine Pumpe erzeugten Druck. Dieses Dokument offenbart keinen Gasmessraum, welcher Gasmessraum in einem ersten Gasmessraum und einen zweiten, räumlich abgeschlossenen oder abschließbaren Gasmessraum mit zumindest einem Gassensor umfasst.

US10492711 betrifft die Impulsoszillometrie.

WO2012014024 betrifft eine Vorrichtung zur Bestimmung von mechanischen Parametern des Respirationstrakts.

DE102006023837 betrifft eine Vorrichtung zur Bestimmung des Alkoholgehaltes in der Lunge unter Beaufschlagung der Lunge mit einem oszillierenden Druck.

WO2008106961 offenbart eine Vorrichtung mit zwei verschließbaren Öffnungen zur Herstellung der Strömungspfade. Beim Einatmen strömt die Atemluft vollständig durch einen Filter mit einer beim Ausatmen geschlossenen Klappe und beim Ausatmen strömt die Atemluft durch ein Peep-Ventil. Dadurch wird ein Druckaufbau durch die Lunge erzwungen. Die Klappe und das Peep-Ventil beeinflussen den Atemvorgang des Patienten.

Die DE20201500382U1 offenbart eine Vorrichtung mit nur einem Gasmessraum mit nur einem Durchströmsensor. Die DE20201500382U1 offenbart getrennte Wege für Einatemluft und Ausatemluft, wobei beim Einatmen die Atemluft vollständig durch den Lufteinlass strömt. Beim Ausatmen strömt die Atemluft vollständig durch den Durchströmsensor, indem diese vom Flowgenerator bei Erreichen eines definierten Drucks abgesaugt wird.

WO2019222464 offenbart keinen abgeschlossenen Gasmessraum.

Die US2018235480A1 offenbart ein Gerät mit einem ersten Strömungspfad, von welchem Luftproben entlang eines zweiten Strömungspfades in eine Messkammer gebracht werden können. In der Messkammer liegt ein Sauerstoff- und/oder ein Kohlenstoffdioxidsensor vor. Im ersten Strömungspfad, welcher dem gegenständlichen ersten Gasmessraum entspricht, liegt kein Gassensor, insbesondere auch kein Kohlenstoffdioxid-Sensor oder Kohlenmonoxid-Sensor vor. Eine Abnehmbarkeit der Messkammer ist nicht beschrieben.

Die WO2017180606A1 offenbart ein Gerät mit einem ersten Strömungspfad, von welchem Luftproben entlang eines zweiten Strömungspfades in eine Messkammer gebracht werden können. In der Messkammer liegt ein Sauerstoff- und/oder ein Kohlenstoffdioxidsensor vor. Im ersten Strömungspfad, welcher dem gegenständlichen ersten Gasmessraum entspricht, liegt kein Kohlenstoffdioxid-Sensor oder Kohlenmonoxid-Sensor vor. Es wird erwähnt, dass im ersten Gasmessraum zusätzliche Sensoren vorliegen können, jedoch handelt es sich bei diesen nicht um einen Kohlenstoffdioxid-Sensor oder Kohlenmonoxid-Sensor. Die Ausbildung des zweiten Strömungspfades als abnehmbaren Gasmessraum ist nicht beschrieben.

Die hier offenbarte Erfindung stellt sich die Aufgabe, eine Vorrichtung zur Ermittlung der Zusammensetzung der Atemluft bereitzustellen. Dies wird durch den Anspruch 1 erreicht.

Die Vorrichtung kann in dem Gasmessraum zumindest einen der folgenden Gassensoren als erster Gassensor und/oder zweiter Gassensor und/oder als weiterer Gassensor zur Bestimmung der relevanten Messwerte umfassen:
Stickstoffmonoxid-Sensor, Kohlenstoffdioxid-Sensor, Sauerstoff-Sensor, Kohlenmonoxid-Sensor, Multi-Gas-Sensor, Sensoren für flüchtige organische Verbindungen, Alkane-Sensor. Erfindungsgemäß fallen nur Ausführungsbeispiele unter die Ansprüche, wenn in dem zweiten Gasmessraum einer der folgenden Gassensoren zur Bestimmung von relevanten Messwerten angeordnet ist: Stickstoffmonoxid-Sensor, Kohlenstoffdioxid-Sensor, Sauerstoff-Sensor, Kohlenmonoxid-Sensor, Multi-Gas-Sensor, Sensor für flüchtige organische Verbindungen, Alkane-Sensor, Alkene-Sensor, oder Aldehyde- Sensor. Erfindungsgemäß fallen nur Ausführungsbeispiele unter die Ansprüche, wenn in dem ersten Gasmessraum einer der folgenden Gassensoren zur Bestimmung von relevanten Messwerten angeordnet ist: Kohlenstoffdioxid-Sensor, Kohlenmonoxid-Sensor.

Die Vorrichtung kann auch einen Gassensor umfassen, welcher Gassensor ähnliche Eigenschaften wie die oben beispielhaft genannten Gassensoren aufweist. Der eingesetzte Gassensor kann allenfalls dazu geeignet sein, zumindest einen Messwert oder zumindest zwei Messwerte der folgenden Messwerte zu bestimmen: Konzentration und/oder Menge von einem Gas aus der folgenden Gruppe: Stickstoffmonoxid, Kohlenstoffdioxid, Sauerstoff, Kohlenmonoxid, Multi-Gas, flüchtige organische Verbindungen (wie Alkane, Alkene oder Aldehyde), Alkane.

Der Fachmann ist in der Lage, unter Heranziehen seines allgemeinen Fachwissens einen zu der hier geschilderten Ermittlung der Messwerte geeigneten Gassensor auszuwählen. Der ausgewählte Gassensor kann insbesondere dazu geeignet sein, mehrere Messwerte in Kombination zu bestimmen.

Der Gassensor kann dazu geeignet sein, die chemischen Bestandteile der Atemluft und/oder das Vorkommen ausgewählter chemischer Bestandteile und/oder die Konzentration ausgewählter chemischer Bestandteile zu ermitteln. Die Vorrichtung kann sich dadurch auszeichnen, dass die mechanischen Messwerte der Lunge und die chemische Zusammensetzung der Atemluft gleichzeitig ermittelbar sind, wobei zwischen den mechanischen Messwerten und der chemischen Zusammensetzung der Atemluft ein synergetischer Effekt feststellbar ist. Die Anwendung der Impulsoszillometrie erlaubt es wichtige Parameter der Lunge oder des Respirationstracks zu ermitteln. Verglichen mit der Spirometrie wird bei dieser Methodik vergleichsweise wenig Mitwirken des Patienten gefordert. Ein Druckimpuls wird hierbei in den Respirationstrakt des Patienten gesendet, worauf der Respirationstrakt reagiert und ebenfalls einen Luftimpuls retour sendet. Diese provozierte Impulsantwort wird über die Druck-Sensorik aufgezeichnet. Aus diesen gewonnenen Messdaten können essenzielle Parameter errechnet werden.

Ein Gassensor nach dem Stand der Technik kann eine Messung auf Basis elektrochemischer Reaktionen und/oder chromatografischer Reaktionen erlauben.

Die hier offenbarte Erfindung stellt sich die Aufgabe, eine Vorrichtung zu einer möglichst genauen Ermittlung von Messwerten beschreibend die Funktion des respiratorischen Systems oder der Lunge eines Patienten bereitzustellen, wobei ein Einfluss auf die Messwerte aus der Bedienung der Vorrichtung, insbesondere das Einbringen der Luft unterbunden werden soll.

Die im Folgenden offenbarte Vorrichtung kann in vorteilhafter Weise die synchrone Durchführung der Spirometrie und der Kapnometrie (CO2-Messung) erlauben. Zusätzlich ist eine Gasanalyse der Atemluft möglich, wobei in einer effizienten Weise die Konzentration von Stickoxiden (NO) und/oder Sauerstoff (O2) und/oder Kohlenstoffmonoxid (CO) gemessen werden kann.

Die Vorrichtung ist zur Früherkennung und Überwachung eines Krankheitsverlaufs von Menschen mit den Krankheiten COPD und/oder Asthma einsetzbar.

Erfindungsgemäß wird dies durch den Anspruch 1 erreicht.

Die Vorrichtung zeichnet sich dadurch aus, dass
der Gasmessraum in einen ersten Gasmessraum und in einen zweiten Gasmessraum räumlich getrennt ist. Diese räumliche Trennung des Gasmessraums in einen ersten Gasmessraum und einen zweiten Gasmessraum ist durch eine verschließbare, den ersten Gasmessraum und den zweiten Gasmessraum verbindende Öffnung zwischen erstem Gasmessraum und zweiten Gasmessraum herstellbar, welche verschließbare Öffnung beim Ausatmen und sohin Einbringen von Atemluft in die Vorrichtung einen ersten Strömungspfad von dem ersten Gasmessraum in den zweiten Gasmessraum und beim Einatmen und sohin Ziehen von Luft aus der Vorrichtung einen zweiten Strömungspfad von der Umgebung über einen Gasmessraumeinlass in den ersten Gasmessraum freigibt.

Es ist sohin der erste Gasmessraum in Strömungsrichtung bei Einbringung von Atemluft in die Vorrichtung vor dem zweiten Gasmessraum angeordnet.

Die verschließbare Öffnung weist zumindest die Eigenschaft auf, dass durch die Öffnung in Schließstellung der zweite Gasmessraum einen Luftraum definiert, welcher Luftraum von der Umgebung und vom ersten Gasmessraum getrennt ist, sodass keine Luft von der Umgebung beziehungsweise von dem ersten Gasmessraum in den zweiten Gasmessraum eindringen kann. Bei einer Anordnung eines Gassensors als zweiter Gassensor in dem zweiten Gasmessraum wirkt ausschließlich der zweite Gasmessraum als Messkammer.

Es ist zumindest ein zweiter Gassensor in dem zweiten Gasmessraum angeordnet. Der Fachmann wählt einen zweiten Gassensor aus den oben erwähnten Gassensoren aus, um eine bestimmte Messung mit der erforderlichen Qualität durchführen zu können.

Der Patient bringt seine Atemluft in einen zweiten Gasmessraum ein, welcher Gasmessraum als ein abgeschlossener oder abschließbarer Raum definiert ist, wobei das räumliche Abschließen des zweiten Gasmessraumes über die verschließbare Öffnung erreicht wird. Durch die Ausbildung des zweiten Gasmessraumes als ein abgeschlossener oder abschließbarer Raum kann das in den zweiten Gasmessraum eingebrachte Luftvolumen, insbesondere die in den zweiten Gasmessraum eingebrachte Atemluft über einen definierten Zeitraum in dem zweiten Gasmessraum gehalten werden. Es kann sohin die Messung mittels des zweiten Gassensors an einem einströmenden und/oder ausströmenden und/oder ruhenden Luftvolumen, insbesondere Atemluft durchgeführt werden.

Es ist ein erster Gassensor in dem ersten Gasmessraum angeordnet, sodass eine Messung mittels dieses Gassensors am strömenden Luftvolumen durchführbar ist. Der Fachmann wählt aus den oben erwähnten Gassensoren einen geeigneten ersten Gassensor aus.

Die Vorrichtung kann auch einen weiteren Gassensor im Mundstück umfassen.

Der Fachmann wählt den weiteren Gassensor aus den oben erwähnten Gassensoren aus, um die gewünschten Messungen in der gewünschten Qualität durchführen zu können.

Das Mundstück kann der gängigen Lehre folgend einen Virenfilter umfassen, welcher Virenfilter das Eindringen von Viren und/oder Bakterien in den Gasmessraum unterbindet. Um dennoch diese Viren und/oder Bakterien in der in die Vorrichtung eingebrachte Atemluft mittels der Vorrichtung ermitteln zu können, kann das Mundstück - in Strömungsrichtung der in die Vorrichtung eingebrachten Atemluft gesehen - vor dem in dem Mundstück angeordneten Filter Virenfilter und/oder Bakterienfilter einen geeigneten Gassensor zur Detektion von in der Atemluft enthaltenen Viren und/oder Bakterien umfassen.

Die Funktionsweise und die mögliche Ausbildung des Gassensors sind bereits oben beschrieben. Der Gassensor kann insbesondere einen Infrarotsensor und/oder Lichtwellensensor und/oder Widerstandssensor und/oder Halbleitersensor umfassen.

Die Vorrichtung kann sich dadurch auszeichnen, dass
der Gassensor einen Trägerkörper mit einer Trägerkörperoberfläche aufweist,
auf welcher Trägerkörperfläche zumindest zwei isolierte Leiterbahnen mit einer Leiterbahnoberfläche angeordnet sind,
auf welcher Leiterbahnoberfläche ein Messkörper umfassend Tetracosane und ein Bindemittel umfassend Acrylic Copolymer, Polyurethane Polymer angeordnet ist.

Der Messkörper wird dem zu messenden Fluid ausgesetzt, wodurch der Messkörper seinen elektrischen Widerstand oder seine elektrische Leitfähigkeit verändert. Bei einer Beaufschlagung der elektrischen Leiterbahn mit elektrischem Strom ist die Veränderung der elektrischen Eigenschaften des Messkörpers ermittelbar.

Der oben beschriebene Gassensor kann so hergestellt werden, dass Acetylene carbon black (CAS No.: 7440-44-0), Tetracosane (CAS No.: 646-31-1) sowie als Bindemittel Acrylic Copolymer, Polyurethane polymer, Ethanol, Wasser, Dimethylether auf die Leiterbahnoberfläche aufgebracht werden.

Das Herstellungsverfahren des Gassensors kann umfassen, dass
8% Acetylene carbon black (CAS No.: 7440-44-0),
12 % Tetracosane (CAS No.: 646-31-1) und
80% des Bindemittels umfassend Acrylic Copolymer, Polyurethane polymer, Ethanol, Wasser, Dimethylether aufgebracht werden,
wobei eine nachfolgende Verdampfung von Acetylene, Ethanol, Wasser, Dimethylether erfolgt.

Der erwähnte Gassensor muss (in Beispielen, die nicht unter die Ansprüche fallen) keinesfalls zwingend in der Vorrichtung vorgesehen sein. Der Gassensor kann auch in anderen Vorrichtungen als in der Vorrichtung vorgesehen sein oder als alleinstehender Gassensor fungieren.

Der Gassensor kann auf verschiedene Bestandteile der Atemluft reagieren. Es seien flüchtige organische Verbindungen wie Alkane und/oder Alkene und/oder Aldehyde erwähnt, auf welche erwähnten Bestandteile dieser Gassensor reagieren kann. Der Gassensor ändert in Abhängigkeit des Vorhandenseins der erwähnten Bestandteile und/oder in Abhängigkeit der Konzentration der erwähnten Bestandteile seine elektrische Leitfähigkeit oder seinen elektrischen Widerstand, was über eine Anspeisung der isolierten Leiterbahn mit elektrischem Strom messbar ist.

Flüchtige organische Verbindungen kommen in der Atemluft von Patienten mit einem Lungenkarzinom häufiger vor als bei gesunden Patienten.

Die Vorrichtung kann das Messen von Luftimpulsmesswerten erlauben, welche Luftimpulsmesswerte das in den Gasmessraum mittels der Pumpvorrichtung und/oder durch Ein- oder Ausatmen eingebrachte Luftimpulsvolumen beschreiben.

Die Vorrichtung kann sich dadurch auszeichnen, dass Drucksensoren beziehungsweise Durchflusssensoren in dem ersten Gasmessraum und/oder in dem zweiten Gasmessraum angeordnet sind, mittels welcher Drucksensoren beziehungsweise Durchflusssensoren Luftimpulsmesswerte beschreibend das in den Gasmessraum durch Ein- oder Ausatmen eingebrachte Luftimpulsvolumen ermittelbar sind. Die direkte Messung der Luftimpulsmesswerte erfolgt über zumindest einen Drucksensor, welcher Drucksensor den Fluiddruck misst. Der Drucksensor kann in dem Gasmessraum angeordnet sein oder in einer Fluidkommunikation mit dem in dem Gasmessraum gehaltenen Fluid stehen. Der Drucksensor kann beispielsweise in dem ersten Gasmessraum angeordnet sein.

Die Bestimmung des in dem Gasmessraum herrschenden Druckes ist auch über indirekte Messmethoden wie beispielsweise das Messen einer Deformation eines Teilbereiches des Gasmessraums durchführbar. Die direkten Messmethoden und die indirekten Messmethoden sind auch kombinierbar.

Die räumliche Trennung des Gasmessraumes in einen ersten Gasmessraum, mit welchem ersten Gasmessraum die Pumpvorrichtung in direkter Fluidkommunikation steht, und in einen zweiten Gasmessraum, welcher zweite Gasmessraum von dem ersten Gasmessraum und von der Pumpvorrichtung und somit von der direkten Wirkung der Pumpvorrichtung durch die verschließbare Öffnung getrennt ist, erlaubt die Anordnung des Drucksensors in dem ersten Gasmessraum und die Anordnung des Gassensors in dem zweiten Gasmessraum. Diese räumlich getrennte Anordnung von Drucksensor und Gassensor erlaubt das effiziente Durchführen von Messungen über die mechanische Funktionalität der Lunge und der chemischen Zusammensetzung der Atemluft.

Die hier offenbarte Erfindung ermöglicht die Bestimmung einer Erkrankung der Lunge oder des Respirationstracks eines Patienten, wobei die Lunge unter Druck im Allgemeinen und/oder Oszillationsdruck im Speziellen besondere Teilmengen einer Atemluft freigibt. Der Fachmann nimmt zum Zeitpunkt des Erstellens dieser Schrift an, dass die Vorrichtung zum Erkennen von Erkrankungen wie COPD, Fibrosen, Asthma, Lungenkrebs, Viren, Bakterien und im Weiteren auch Entzündung von Teilbereichen der Atemwege und/oder Vorstadien dieser Erkrankungen geeignet ist. Es wird kein Anspruch auf Vollständigkeit der hier beispielhaft angegebenen Liste der mittels der Vorrichtung ermittelbaren Krankheiten erhoben; der Fachmann kennt weitere Krankheiten mit zu den beispielhaft erwähnten Krankheiten ähnlichen Symptomen.

Es gibt beispielsweise erste Indizien, dass Lungenkrebs unter anderem mit Hilfe von Sensoren für flüchtige organische Verbindungen ermittelbar ist. Es wird zur Durchführung einer solchen Untersuchung eine Atemluft auf den Sensor für flüchtige organische Verbindungen aufgebracht, sodass die zu untersuchende Atemluft den Sensor umgibt. Die Vorrichtung kann - wie oben angegeben - einen Sensor für flüchtige organische Verbindungen als Gassensor umfassen.

Im Zuge der Entwicklung der hier diskutierten Vorrichtungen wurde auch die Effizienz und/oder die Aussagekraft und/oder die Genauigkeit von Messungen mit der Vorrichtung untersucht. Es zeigt sich, dass Kohlenmonoxid-Sensoren, welche Kohlenmonoxid-Sensoren in der Vorrichtung angeordnet sind, eine genauere Bestimmung von metabolischen Kohlenstoffen in der auf den Sensor aufgebrachten Atemluft und/oder auch eine Bestimmung von kleineren Mengen von metabolischen Kohlenstoffen in der auf den Sensor aufgebrachten Atemluft als beispielsweise ein Alkanesensor erlauben. Dies erlaubt eine genauere und/oder effizientere Bestimmung von Lungenkrebs.

Die hier beschriebene genauere und/oder effizientere Bestimmung von Lungenkrebs kann dadurch begründet sein, dass der in der Vorrichtung angeordnete Kohlenmonoxid-Sensor nicht ausschließlich zur Ermittlung eines Vorkommens von Kohlenmonoxid geeignet ist.

Die Vorrichtung kann sich dadurch auszeichnen, dass
die Vorrichtung eine Pumpvorrichtung umfasst, welche Pumpvorrichtung zum Einbringen eines Luftimpulsvolumens in den Gasmessraum mit dem Gasmessraum in Fluidkommunikation steht.

Die Pumpvorrichtung verursacht eine Bewegung und/oder eine Komprimierung eines im Gasmessraum gehaltenen Fluids. Dies kann so bewerkstelligt werden, dass hierzu ein Kolben bewegt oder ein den Gasmessraum definierender Behälter deformiert wird. Die Bewegung des Kolbens und/oder die Deformation des Gasmessraumes, insbesondere einer Hülle des Gasmessraumes erfolgt vorzugsweise oszillierend.

Bei einer Anordnung von Drucksensoren oder Durchflusssensoren in dem Gasmessraum können die Luftimpulswerte beschreibend das mittels der Pumpvorrichtung in den Gasmessraum eingebrachte Luftimpulsvolumen ermittelt werden.

Der Drucksensor und/oder der Druchflußsensor kann in dem ersten Gasmessraum angeordnet sein, sodass mittels des im ersten Gasmessraum angeordneten Drucksensors und/oder Durchflußsensors der durch die Pumpvorrichtung erzeugte Luftimpuls anhand von Luftimpulsmesswerten bestimmbar ist.

Die hier offenbarte Vorrichtung kann sohin auf einer Kombination von einem Spirometer oder einem Impulsoszillometer mit einer Gasmessvorrichtung basieren.

Es können mehrere Gassensoren in dem Gasmessraum angeordnet sein. Der Fachmann kann eine Anzahl von Gassensoren zur Durchführung einer bestimmten Anzahl von Messungen über einen bestimmten Zeitraum auswählen.

Die Pumpvorrichtung kann einen Membrankompressor umfassen,
welcher Membrankompressor ein an den Gasmessraum angeschlossenes Kompressorvolumen umfasst, welches Kompressorvolumen durch eine in eine Membranschwingung versetzbare Membran komprimierbar ist.

Eine mögliche Ausführungsform der Pumpvorrichtung ist ein Lautsprecher oder eine einem Lautsprecher ähnliche Vorrichtung, welcher vorzugsweise unter Ausbildung eines fluiddichten Bereiches mit dem Gasmessraum in Fluidverbindung steht. Die Ausbildung des fluiddichten Bereiches kann auf bestimmte Fluiddrücke definiert sein.

Die Vorrichtung kann sich dadurch auszeichnen, dass
die Membranschwingung 1-50 Hz, vorzugsweise 1-32 Hz beträgt, in speziellen Fällen aber auch höherfrequent betrieben werden kann.

Die Pumpvorrichtung kann eine Drehschieberpumpe mit einem Rotor und/oder eine Kolbenpumpe mit einem Kolben umfassen,
welche Pumpvorrichtung ein an dem Gasmessraum angeschlossenes Kompressorvolumen umfasst, welches Kompressorvolumen durch eine Rotation des Rotors beziehungsweise durch eine Bewegung des Kolbens komprimierbar ist.

Es kann mittels der Pumpvorrichtung ein Verschiebevolumen von maximal 100,0 Milliliter, vorzugsweise 40,0 Milliliter über die dritte Öffnung in den Gasmessraum eingebracht werden.

Die angegebenen Frequenzen und das angegebene Verschiebevolumen sind die bei einer Impulsoszillometrie üblicher Weise eingesetzten Frequenzen. Der Fachmann kann eine andere Frequenz oder ein anderes Verschiebevolumen durch eine geänderte Dimensionierung der Pumpvorrichtung vorsehen, um dem Respirationstrakt des Patienten die Abgabe von besonderen Teilmengen der Atemluft zu provozieren. Die oben angegebenen Frequenzen und das oben angegebene Verschiebevolumen wurde bei Testverfahren angewandt.

Die Ausbildung des Kolbens der Pumpvorrichtung als Membran, welche Membran in eine Schwingung versetzt oder in eine Endlage bewegt wird, hat den Vorteil, dass mit einer relativ kleinen Masse ein relativ großes Fluidvolumen bewegt werden kann. Es können sohin störende Vibrationen in der Vorrichtung vermieden werden.

Die verschließbare Öffnung kann als ein Ventil und/oder ein Rückschlagventil ausgebildet sein. Der Fachmann kann auch weitere verschließbare Öffnungen nach dem Stand der Technik vorsehen, um den zweiten Gasmessraum als einen abgeschlossenen oder abschließbaren Raum auszubilden. Der Fachmann kann auch eine durch einen Antrieb und/oder eine Steuerung verschließbare Öffnung vorsehen.

Die verschließbare Öffnung kann ergänzend oder alternativ zu der Ausbildung als ein Ventil und/oder als ein Rückschlagventil eine Gasmessraum-Pumpvorrichtung umfassen.

Nach dem Stand der Technik umfasst eine Pumpvorrichtung im Allgemeinen einen Kolben, welcher Kolben in einem Kolbenraum bewegbar ist. Die Vorrichtung kann sich dadurch auszeichnen, dass der bewegbare Kolben zum Öffnen oder Schließen der verschließbaren Öffnung verwendet wird. Der Kolben wird hierzu in eine einen Durchfluss zwischen dem ersten Gasmessraum und dem zweiten Gasmessraum freigebende Stellung oder in eine diesen Durchfluss unterbindende Stellung gebracht.

Die Ausbildung der verschließbaren Öffnung als eine Gasmessraum-Pumpvorrichtung erlaubt weiters das Einbringen einer definierten Luftmenge vom ersten Gasmessraum in den zweiten Gasmessraum. Nach dem Stand der Technik ist diese Luftmenge durch die Bewegung des Kolbens definierbar. Durch das Einbringen einer definierten Luftmenge in den zweiten Gasmessraum ist die Qualität der Messung mittels des zweiten Gassensors erhöhbar. Das Beaufschlagen des Gassensors mittels einer definierten Luftmenge hat insbesondere den Vorteil, dass die Messung in einer bestimmten Qualität wiederholbar ist.

Die Vorrichtung kann sich dadurch auszeichnen, dass
die verschließbare Öffnung beim Einatmen und sohin Ziehen von Luft aus der Vorrichtung einen zweiten Strömungspfad von einem mit der Umgebung in Fluidkommunikation stehenden Gasmessraumeinlass in den ersten Gasmessraum freigibt.

Der Fachmann kann dies durch die Anordnung eines Ventils und/oder eines Rückschlagventils und/oder einer Gasmessraum-Pumpvorrichtung in der verschließbaren Öffnung erreichen.

Wenngleich die mechanische Funktionalität der Lunge oder des respiratorischen Systems und die chemische Zusammensetzung der Atemluft synergetische Effekte aufweisen, so können durch die oben beschriebene räumlich getrennte Anordnung der Messmittel wie Gassensor, Drucksensoren, Durchflusssensoren Messungen durchgeführt werden, welche Messungen die Eigenschaften des Drucksensors und des Gassensors vor der Messung, während der Messung und nach der Durchführung der Messung berücksichtigen.

Die mechanische Funktionalität der Lunge wird durch die Drucksensoren ermittelt. Diese Messung dauert im Regelfall nicht lange, da ein Drucksensor in der Regel rasch reagiert und keine oder nur eine geringe Ausgleichszeit braucht.

Die erforderliche Messung zur Bestimmung der chemischen Zusammensetzung der Atemluft wird in dem zweiten oder einem der Gasmessräume durchgeführt. Ein Gassensor nach dem Stand der Technik benötigt nach der Beaufschlagung mit der Atemluft eine Ausgleichsphase. Die räumliche Trennung in den ersten Gasmessraum und den zweiten Gasmessraum hat den Vorteil, dass der Patient ausschließlich über den ersten Gasmessraum einatmen kann und dem in dem zweiten Gasmessraum befindlichen zweiten Gassensoren eine ausreichende Zeitspanne zum Ausgleich gewährt wird.

Die Vorrichtung kann sich dadurch auszeichnen, dass das Mundstück einen Virenfilter umfasst.

Zur Bestimmung von Viren und/oder Bakterien in der Atemluft mittels der Gassensoren verwendet der Fachmann ein filterfreies Mundstück.

Die Vorrichtung kann sich dadurch auszeichnen, dass das Mundstück einen Luftentfeuchter umfasst.

Die Feuchtigkeit der ausgeatmeten Luft ist sehr hoch. Um Messfehler und auch eine Beschädigung der Vorrichtung zu unterbinden, sollte die Flüssigkeit aus der Atemluft möglichst vollständig entfernt werden. Es soll im Wesentlichen die Luftfeuchtigkeit in der Atemluft soweit gesenkt werden, dass sich in der Messeinrichtung kein Kondensat bilden kann. Der Fachmann kann eine solche maximale Luftfeuchtigkeit in der Atemluft mit seinem Fachwissen abschätzen.

Der Virenfilter und der Luftentfeuchter können einstückig ausgebildet sein.

Das Mundstück, insbesondere der einstückig ausgebildete Virenfilter und Entfeuchter kann nach dem Einbringen von Atemluft in die Vorrichtung oder einer gewissen Anzahl des Einbringens von Atemluft in die Vorrichtung ausgetauscht werden.

Die Vorrichtung kann sich dadurch auszeichnen, dass die Vorrichtung einen in dem ersten Gasmessraum angeordneten ersten Temperatursensor und/oder einen in dem zweiten Gasmessraum angeordneten zweiten Temperatursensor umfasst.

Mit Hilfe des ersten Temperatursensors ist die Temperatur der Atemluft im ersten Gasmessraum ermittelbar. Der erste Temperatursensor kann hierzu in einer unmittelbaren Nähe zu dem Drucksensor angeordnet sein, sofern die Vorrichtung einen solchen Drucksensor umfasst.

Mittels des zweiten Temperatursensors ist die Temperatur der Atemluft im zweiten Gasmessraum ermittelbar. Der zweite Temperatursensor kann hierzu in unmittelbarer Nähe zu dem Gassensor angeordnet sein, sodass die Temperatur über die Dauer der Gasmessung und/oder des Ausgleiches des Gassensors ermittelbar ist. Über den zweiten Temperatursensor ist auch gegebenenfalls der Status des Ausgleiches des Gassensors ermittelbar.

Bei einer Anordnung von dem ersten Temperatursensor und dem zweiten Temperatursensor ist auch eine Temperaturdifferenz ermittelbar.

Die Vorrichtung kann einen in dem ersten Gasmessraum angeordneten ersten Feuchtesensor und/oder einen in dem zweiten Gasmessraum angeordneten zweiten Feuchtesensor umfassen.

In Analogie zu der oben angeführten Anordnung der Temperatursensoren können die Feuchtesensoren in unmittelbarer Nähe zu dem Drucksensor beziehungsweise zu dem Gassensor angeordnet sein. Der Fachmann kann auch Sensoren vorsehen, über welche Sensoren eine Temperatur der Atemluft und ein Feuchtegehalt der Atemluft in Kombination ermittelbar ist. Bei einer Anordnung eines ersten Feuchtesensors und eines zweiten Feuchtesensors ist gegebenenfalls eine Feuchtedifferenz ermittelbar. Über die mittels des zweiten Feuchtesensors ermittelbare Feuchte ist der Ausgleich des Gassensors ermittelbar.

Die Vorrichtung kann
- einen Drucksensor zur Bestimmung des atmosphärischen Umgebungsdruckes der Umgebung der Vorrichtung und/oder
- einen Gassensor zur Bestimmung der Gaszusammensetzung des Umgebungsgases der Umgebung der Vorrichtung und/oder
- einen Temperatursensor zur Bestimmung der Umgebungstemperatur der Umgebung der Vorrichtung und/oder
- einen Feuchtigkeitssensor zur Bestimmung der Umgebungsfeuchte der Umgebung der Vorrichtung umfassen.

Die oben angegebenen Sensoren sind zur Ermittlung von Parametern geeignet, mittels welcher Parameter der Zustand der Umgebung der Vorrichtung beschreibbar ist. Der Fachmann erkennt, dass diese Sensoren hierzu zumindest in einer Wechselwirkung zu der Umgebung der Vorrichtung stehen müssen, um eine derartige Messung durchführen zu können. Diese Sensoren können auch in der Umgebung angeordnet sein. Vorzugsweise sind diese Sensoren an den Außenoberflächen der Vorrichtung angeordnet.

Es kann die Pumpvorrichtung in Abhängigkeit des atmosphärischen Umgebungsdruckes gesteuert werden. Die Pumpvorrichtung kann beispielsweise einen Druck im ersten Gasmessraum und sohin einen Fluiddruck in der Lunge des Patienten erzeugen, welcher Druck und welcher Fluiddruck in der Lunge sich um einen Differenzdruck von dem atmosphärischen Umgebungsdruck unterscheidet. Der Differenzdruck kann ein vorgegebener Wert sein oder in Abhängigkeit des atmosphärischen Umgebungsdruckes und/oder der zu ermittelten Krankheit festgelegt werden.

Die Vorrichtung kann sich dadurch auszeichnen, dass über den Gasmessraumeinlass Substanzen mit einer medizinischen Wirkung, eine Flüssigkeit, eine Verunreinigung der Luft einbringbar sind.

Die in die Vorrichtung eingebrachten medizinischen Substanzen können eine Wirkung auf die Atemwege, insbesondere die Lunge und somit das Verhalten der Atemwege, insbesondere der Lunge haben. Der Fachmann kennt beispielsweise Asthmamittel und Mittel zur spezifischen und unspezifischen bronchialen Provokation, welche Mittel der Fachmann als medizinische Substanzen über den Gasmessraumeinlass in die Vorrichtung und über die Vorrichtung in die Atemwege des Patienten einbringen kann.

Der Fachmann kann eine Flüssigkeit zur Anreicherung der Luft in die Vorrichtung einbringen.

Der Fachmann kann auch eine Verunreinigung der Luft gezielt in die über die Vorrichtung gezogene Luft einbringen.

Die Vorrichtung kann sich dadurch auszeichnen, dass die eingebrachten medizinischen Substanzen und/oder die eingebrachte Flüssigkeit und/oder die eingebrachte Verunreinigung mittels der Gassensoren analysiert werden können. Die Einbringung in die Atemwege des Patienten kann über die Pumpvorrichtung verstärkt werden, was wiederum mittels der Drucksensoren messbar und kontrollierbar ist.

Die Vorrichtung kann eine im Gasmessraumeinlass angeordnete Heizvorrichtung und/oder eine ebendort angeordnete Kühlvorrichtung zum Heizen beziehungsweise Kühlen der in den Gasmessraum eingebrachten Luft und/oder der Sensoren umfassen. Es kann sohin die Temperatur der in die Atemwege des Patienten eingebrachten Luft gesteuert werden und sohin eine Reaktion der Atemwege, insbesondere der Lunge auf eine eingebrachte Luft mit einer bestimmten Temperatur ermittelt werden.

Die verwendeten Sensoren wie Gasmesssensoren, Temperatursensoren, Drucksensoren et cetera können bei einem bestimmten Temperaturbereich eine besonders hohe Messgenauigkeit aufweisen. Aus diesem Grund kann es erforderlich sein, Teile der Vorrichtung wie beispielsweise die erwähnten Sensoren oder die gesamte Vorrichtung auf einen bestimmten Temperaturbereich zu erwärmen beziehungsweise zu kühlen. Der Fachmann wählt diesen Temperaturbereich in Abhängigkeit der Eigenschaften der eingesetzten Sensoren.

In vorteilhafter Weise werden jene Teile der Vorrichtung erwärmt, welche Teile der Vorrichtung von der eingebrachten Atemluft umspült werden.

Die Vorrichtung kann sich dadurch auszeichnen, dass die Vorrichtung ein Datenkommunikationsmittel zur Übertragung der mittels des Sensors ermittelten Daten an ein Datenverarbeitungsgerät umfasst.

Die Vorrichtung hat die Funktion der Ermittlung der Messwerte mittels der erwähnten Gassensoren und/oder sonstigen Messvorrichtungen. Es sind oben beispielsweise Drucksensoren, Durchflusssensoren, Feuchtesensoren als weitere Messvorrichtungen angeführt.

Die Vorrichtung kann auf die Ermittlung der Messwerte und auf die Übertragung dieser Messwerte an ein Datenverarbeitungsgerät beschränkt sein.

Die Vorrichtung kann sich dadurch auszeichnen, dass die Vorrichtung ein Zählwerk umfasst.

Die Qualität der mittels eines Gassensors durchgeführten Messung kann mit der Anzahl der durchgeführten Messungen im speziellen und/oder mit der Anzahl des Einbringens von Atemluft in die Vorrichtung abnehmen. Die angeführte Abnahme der Messqualität ist bei Gassensoren im Allgemeinen und im Speziellen bei den oben erwähnten Gassensoren, welche Gassensoren als erste Gassensoren und/oder zweite Gassensoren und/oder weitere Gassensoren in der Vorrichtung umfasst sind, zu beobachten.

Mit Hilfe des Zählwerkes ist ermittelbar, wie oft ein Patient oder Patienten Atemluft in die Vorrichtung einbringen und/oder wie oft eine Atemluft auf einen Gassensor aufgebracht wird. Die Aufbringung der Atemluft auf einen Gassensor kann unabhängig von der Durchführung einer Messung mit einem Gassensor erfolgen, das heißt es kann eine Atemluft auf den Gassensor aufgebracht werden und eine Gasmessung oder keine Gasmessung durchgeführt werden.

Das Zählwerk kann in der Vorrichtung und/oder in dem Datenverarbeitungsgerät integriert sein.

Die Vorrichtung, insbesondere die Sensoren der Vorrichtung können bei Erreichen eines Grenzwertes von einer durchgeführten Beaufschlagung des Gassensors mit Atemluft und/oder einer Verwendungsanzahl der Vorrichtung ausgetauscht werden.

Die Möglichkeit des Austauschens der Vorrichtung oder der Sensoren kann den Vorteil haben, dass bestimmte Sensoren, insbesondere bestimmte Gassensoren ausgewählt werden. Weiters kann ein Softwareupdate durchgeführt werden.

Die hier beschriebene Vorrichtung kann ein tragbares Gerät sein. Die Vorrichtung kann in einem Verfahren, welches nicht unter die Ansprüche fällt, zur Bestimmung zumindest einer der folgenden Krankheiten verwendet werden: Entzündung der Lunge, COPD, Fibrosen, Asthma, Lungenkrebs und Entzündung von Teilbereichen der Atemwege des Patienten.

Das Verfahren kann auch umfassen, dass mehrere Differenzdrücke eines Differenzdruckbereiches beispielsweise zum Erkennen mehrerer Krankheiten mittels der Pumpvorrichtung erzeugt werden.

Das Verfahren kann umfassen, dass die erwähnten Sensoren, insbesondere die erwähnten Gassensoren erwärmt werden.

Das Verfahren kann ein computerimplementiertes Verfahren sein, welches auf dem Vergleich von Mustern von Messwerten basiert. Es können beispielsweise die Muster von Messwerten einer gesunden Person und die Muster von Messwerten einer erkrankten Person angewandt werden. Weiters können die Muster von Messwerten einer Person bei einer Messung zu einem ersten Zeitpunkt und die Muster von Messwerten dieser Person oder einer weiteren Person bei einer Messung zu einem zweiten Zeitpunkt verglichen werden. Die Vorrichtung ist insbesondere zur Beobachtung einer Person unter Quarantäne geeignet.

Der Fachmann erkennt, dass hierbei Methoden nach dem Stand der Technik zu Datenanalyse wie beispielsweis Verfahren der künstlichen Intelligenz eingesetzt werden können.

Die Erfindung wird anhand der folgenden, in den Figuren dargestellten Ausführungsformen ergänzend erläutert:
Figur 1 zeigt eine schematische Darstellung einer Ausführungsform der Vorrichtung.
Figur 2 zeigt eine dreidimensionale Schnittansicht der in Figur 1 schematisch dargestellten Ausführungsform der Vorrichtung.

Die in den Figuren gezeigten Ausführungsformen zeigen lediglich mögliche Ausführungsformen, wobei an dieser Stelle bemerkt sei, dass die Erfindung nicht auf diese speziell dargestellten Ausführungsvarianten derselben eingeschränkt ist, sondern auch Kombinationen der einzelnen Ausführungsvarianten untereinander und eine Kombination einer Ausführungsform mit der oben angeführten allgemeinen Beschreibung möglich sind. Diese weiteren möglichen Kombinationen müssen nicht explizit erwähnt sein, da diese weiteren möglichen Kombinationen aufgrund der Lehre zum technischen Handeln durch gegenständliche Erfindung im Können des auf diesem technischen Gebiet tätigen Fachmannes liegen.

Der Schutzbereich ist durch die Ansprüche bestimmt.

In den Figuren sind die folgenden Elemente durch die vorangestellten Bezugszeichen gekennzeichnet:
- 1: Mundstück
- 2: erster Gasmessraum
- 3: zweiter Gasmessraum
- 4: Ventil
- 5: Gasmessraumeinlass, Lüftungsgitter
- 6: Pumpvorrichtung
- 10: freies Ende des Mundstückes 1
- 11: Scheibe
- 12: Erstreckungsachse
- 13: angeschlossenes Ende des Mundstückes 1
- 21: erste Öffnung des Gasmessraumes
- 22: erster Strömungspfad
- 23: zweiter Strömungspfad
- 24: zweite Öffnung des Gasmessraumes
- 25: dritte Öffnung des Gasmessraumes
- 26: Pumpbehälter
- 27: Kolben
- 28: Gasmessraumauslass, vierte Öffnung
- 29: Schaltflanke
- 30: Ladestecker
- 31: Steuerungseinheit
- 32: Datenkommunikationsmittel
- 33: Gasmessraum-Pumpvorrichtung
- 34: Dichtung
- 35: Gasmessteilraum
- 36: erster Drucksensor
- 37: erster Emitter
- 38: erster Detektor
- 39: Rückschlagventil
- 40: zweiter Drucksensor
- 41: zweiter Gasmesssensor

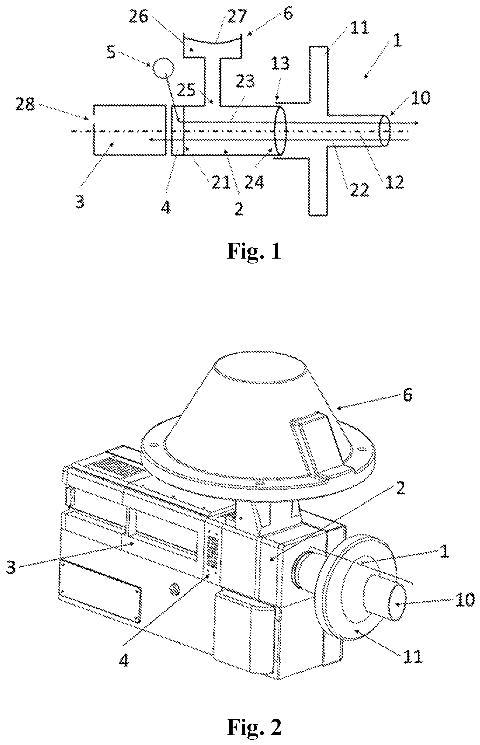

Figur 1 zeigt eine mögliche Ausführungsform der Vorrichtung, wobei in Figur 1 lediglich die funktionellen Elemente der Vorrichtung dargestellt sind.

Ein Patient bringt über ein Mundstück 1 seine Atemluft in die Vorrichtung ein. Das Mundstück 1 bildet einen durch ein Rohr ausgebildeten Fluidkanal aus, welcher Fluidkanal sich vom freien Ende 10 des Mundstückes 1 bis zu dem angeschlossenen Ende 13 des Mundstückes 1 erstreckt. Der Fluidkanal wird durch das Mundstück 1 definiert und erstreckt sich im Wesentlichen geradlinig.

Der Patient umschließt hierzu mit seinen Lippen das freie Ende 10 des Mundstückes 1. Das freie Ende 10 des Mundstückes 1 weist eine nach dem einschlägigen Stand der Technik bekannte Form oder eine aus dem bekannten Stand der Technik ableitbare Form auf. Das freie Ende 10 umfasst eine Öffnung mit einem kreisrunden oder elliptischen Querschnitt, wobei auch andere Formen denkbar sind. Es ist durch den offenen Querschnitt des freien Endes 10 eindeutig ein Querschnitt mit einer Querschnittsgröße definiert, über welchen Querschnitt beim Ausatmen die Atemluft in die Vorrichtung eingebracht wird.

Das Mundstück 1 umfasst weiters eine Scheibe 11, welche Scheibe 11 als Distanzelement dient. Den Stutzen vor der Scheibe, in Richtung des freien Endes 10, umschließt der Patient fest mit seinen Lippen (in Figur 1 nicht dargestellt). Somit entsteht eine luftdichte Verbindung zwischen der Vorrichtung und dem Patienten.

Die Scheibe 11 weist eine Scheibenfläche auf, welche Scheibenfläche im Wesentlichen normal zu der Erstreckungsachse 12 des Mundstückes 1 orientiert ist. Die Abdichtung zwischen Mundstück 1 und den Lippen kann eine wesentliche Voraussetzung zu einer effizienten Einbringung von Fluiddruck in die Lunge des Patienten sein, sodass über einen durch den Fluiddruck erzeugten Lungendruck bestimmte Teilmengen an Atemluft aus der Lunge abgebbar sind.

Das Mundstück 1 kann einen Filter umfassen, welcher Filter im Inneren des Mundstückes 1 angeordnet ist. Der Filter kann dazu geeignet sein, ausgewählte Bakterien und/oder ausgewählte Viren zu filtern. Der Filter kann auch dazu geeignet sein, im Wesentlichen alle nach der gängigen Lehre bekannten Bakterien und/oder im Wesentlichen alle nach der gängigen Lehre bekannten Viren zu filtern. Der Filter kann auch dazu geeignet sein, die in der eingebrachten Atemluft enthaltene Flüssigkeit aus der Atemluft zu entfernen; der Filter kann sohin als Entfeuchter wirken.

Wenn mittels der Vorrichtung das Vorkommen von Viren und/oder Bakterien in der Atemluft bestimmt werden soll, verwendet der Fachmann ein Mundstück 1 ohne Filter.

Es kann auch im Mundstück 1 ein weiterer Gassensor zur Ermittlung von Messwerten beschreibend die in die Vorrichtung eingebrachte Atemluft angeordnet sein.

Der Fachmann kann beispielsweise ein nach dem Stand der Technik bekanntes Mundstück 1 wie Care Fusion gegebenenfalls mit dem Filter MICROGARD ^{®} II TYPE B, BACTERIAL / VIRAL FILTER / V-892380 vorsehen. Der Filter hat den Effekt, dass keine für weitere Patienten schädlichen Bakterien und/oder Viren in die Vorrichtung eingebracht werden und durch die weiteren Patienten aufgenommen werden können.

Der Filter kann im Inneren der Scheibe 11 angeordnet sein.

Das Mundstück 1 kann an seinem freien Ende 10 einen kleineren Querschnitt als an seinem angeschlossenen Ende 13 aufweisen.

Das angeschlossene Ende 13 des Mundstückes 1 ist an einem ersten Gasmessraum 2 angeschlossen. Das Mundstück 1 ist abnehmbar angeschlossen, wobei bei der in Figur 1 dargestellten Ausführungsform das Mundstück 1 in einfacher Weise auf ein Einlassrohr der ersten Gasmessraum 2 aufgeschoben wird. Die genaue Ausbildung des Anschlusses des Mundstückes 1 an dem ersten Gasmessraum 2 hat keinen wesentlichen Einfluss auf die Vorrichtung, sofern der Anschluss eine Fluidkommunikation erlaubt und gegenüber der Umgebung dicht abschließt.

Die im Filter angesammelten Bakterien und/oder Viren können sohin einem weiteren Patienten nicht schaden. Es wird stets ein Mundstück 1 für nur einen Patienten verwendet.

Es ist an einer ersten Öffnung 21 des ersten Gasmessraumes 2 ein Ventil 4 angeordnet, welches Ventil 4 beim Ausatmen und sohin bei einem Einbringen von Atemluft in den ersten Gasmessraum 2 die Atemluft in einen zweiten Gasmessraum 3 weiterleitet (erster Strömungspfad 22). Beim Einatmen oder beim Ansaugen von Luft aus der Vorrichtung durch den Patienten wird die Luft über einen gegebenenfalls mit Lüftungsschlitzen versehenen Gasmessraumeinlass 5 angesaugt (zweiter Strömungspfad 23). Das Ventil 4 schaltet sohin in Abhängigkeit der Strömungsrichtung im ersten Gasmessraum 2 die Fluidkommunikation zwischen dem zweiten Gasmessraum 3 und dem Mundstück 1 (erster Strömungspfad 22) sowie dem Gasmessraumeinlass 5 und dem Mundstück 1 andererseits (zweiter Strömungspfad 23). Das Ventil 4 ist im Schnittbereich zwischen dem ersten Gasmessraum 2, den Lüftungsgittern 5 und dem zweiten Gasmessraum 3 angeordnet.

Das Ventil 4, welches Ventil 4 eine den ersten Gasmessraum 2 und den zweiten Gasmessraum 3 verbindende, verschließbare Öffnung ausbildet, kann beispielsweise als ein Rückschlagventil ausgebildet sein. Das Rückschlagventil kann als Membranrückschlagventil ausgeführt sein, wobei der Fachmann mit seinem Fachwissen auch ein anderes Mittel hierzu vorsehen kann.

Es ist an einer zweiten Öffnung 24 des ersten Gasmessraums 2 das Mundstück 1 angeschlossen. Es wird sohin über die zweite Öffnung 24 die Fluidkommunikation zwischen dem Mundstück 1 und dem ersten Gasmessraum 2 hergestellt. Die zweite Öffnung 24 geht in das Einlassrohr über, auf welches Einlassrohr das Mundstück 1 aufgeschoben wird. Figur 1 zeigt den Sonderfall, dass das Mundstück 1 direkt an dem ersten Gasmessraum 2 angeschlossen ist. Es kann auch zwischen dem ersten Gasmessraum 2 und dem Mundstück 1 ein weiteres Element unter Wahrung der Fluidkommunikation zwischen dem ersten Gasmessraum 2 und dem Mundstück 1 angeschlossen sein. Über das weitere Element kann beispielsweise der Winkel des Mundstückes 1 zu dem ersten Gasmessraum 2 eingestellt werden.

Es kann an einer dritten Öffnung 25 des ersten Gasmessraums 2 in Fluidkommunikation mit dem ersten Gasmessraum 2 eine Pumpvorrichtung 6 angeschlossen sein. Mittels der Pumpvorrichtung 6 können die in dem ersten Gasmessraum 2 herrschenden Fluiddrücke gesteuert werden. Die Pumpvorrichtung 6 kann in dem ersten Gasmessraum 2 sowohl einen Überdruck als auch einen Unterdruck erzeugen. Der Fachmann erkennt, dass der in der ersten Gasmessraum 2 erzeugte temporäre Unterdruck oder Überdruck bei einer über das Mundstück 1 hergestellten Fluidkommunikation mit dem Mundraum des Patienten einen Einfluss auf die Lunge des Patienten hat, zumal der Patient mit seinen Lippen das freie Ende 10 des Mundstückes 1 dichtend umschließt.

Mit Hilfe des Druckmessmittels ist nach dem Stand der Technik in dem ersten Gasmessraum 2 eine Pumpvorrichtung 6 angeschlossen.

Die Figur 1 zeigt den Sonderfall, dass die Pumpvorrichtung 6 direkt an dem ersten Gasmessraum 2 angeschlossen ist. Die Pumpvorrichtung 6 umfasst einen Pumpbehälter 26, wobei das im Pumpbehälter 26 enthaltene Fluidvolumen durch eine Bewegung des Kolbens 27 komprimierbar oder dekomprimierbar ist. Es kann in dem ersten Gasmessraum 2 ein Drucksensor angeordnet sein. Mit Hilfe des in dem ersten Gasmessraum 2 angeordneten Drucksensors können in dem ersten Gasmessraum 2 herrschende Fluiddrücke zufolge Einatmen durch den Patienten oder Ausatmen des Patienten oder zufolge einer durch die Pumpvorrichtung 6 hervorgerufenen Änderung des Fluiddruckes bestimmbar sein. Die Fluiddruckänderungen zufolge Einatmen oder Ausatmen sind auch in Kombination mit der Fluiddruckänderung durch die Pumpvorrichtung 6 messbar.

Mittels der in dem ersten Gasmessraum 2 angeordneten Drucksensoren ist der über einen Zeitraum andauernde Fluiddruck messbar. Es sind weiters Luftimpulsmesswerte ermittelbar, welche Luftimpulsmesswerte das in dem ersten Gasmessraum 2 mittels der Pumpvorrichtung 6 eingebrachte Luftimpulsvolumen oder das durch Einatmen oder Ausatmen gegebenenfalls in Kombination mit der Pumpvorrichtung 6 eingebrachte Luftimpulsvolumen beschreiben.

Bei einer Testvorrichtung wurden BMP280 Sensoren von Bosch als Drucksensoren eingesetzt.

Anstelle des Drucksensors oder in Ergänzung zu dem Drucksensor kann auch ein Strömungssensor in dem ersten Gasmessraum 2 angeordnet sein, über welchen Strömungssensor die Strömungsgeschwindigkeit des Fluids in dem ersten Gasmessraum 2 ermittelbar ist. Nach dem physikalischen Gesetz von Hagen-Poiseuille wird festgehalten, dass bei laminarer Strömung aus dem Fluiddruck die Strömungsgeschwindigkeit und umgekehrt ermittelbar ist.

Der Fachmann erkennt, dass beim Messen einer ausschließlich durch Einatmen und Ausatmen verursachten Fluiddruckänderung in dem ersten Gasmessraum 2 der Kolben 27 in eine definierte Lage zu bringen ist.

Es sind in dem zweiten Gasmessraum 3 zweite Gassensoren zur Bestimmung des in dem zweiten Gasmessraum 3 enthaltenen Fluids angeordnet. Mittels der zweiten Gassensoren wird sohin das in dem zweiten Gasmessraum 3 enthaltene Fluid in Hinblick auf die chemischen Bestandteile des Fluids untersucht. Die Detaillierung einer solche Untersuchung hängt von den eingesetzten Gassensoren ab.

Die in Figur 1 gezeigte Ausführungsform der Vorrichtung zeichnet sich sohin dadurch aus, dass die Drucksensoren zur Bestimmung der Fluiddrücke in dem ersten Gasmessraum 2 und die zweiten Gassensoren zur Bestimmung der Bestandteile des Fluids in dem zweiten Gasmessraum 3 angeordnet sind. Die Drucksensoren und die zweiten Gassensoren sind sohin räumlich getrennt. Bei der in Figur 1 gezeigten Ausführungsform wird diese räumliche Trennung durch das Ventil 4 bewerkstelligt. Der Fachmann kann in Ergänzung oder alternativ zu dem Ventil 4 auch weitere Trennmittel vorsehen, welche Trennmittel beispielsweise auf dem temporären Verschließen einer Öffnung oder eines Kanals, welche Öffnung oder welcher Kanal den ersten Gasmessraum 2 und den zweiten Gasmessraum 3 verbindet. Bei dem anhand von Figur 1 diskutierten Ausführungsbeispiel wirkt das Ventil 4 als temporäres Trennmittel.

Mittels der zweiten Gassensoren ist lediglich die ausgeatmete, d.h. die durch Ausatmen in die Vorrichtung eingebrachte Atemluft zu bestimmen. Die beim Einatmen in die Vorrichtung eingebrachte Luft muss nicht zwingend mittels der Gassensoren bestimmt werden, da die Bestandteile dieser Luft im Wesentlichen bekannt sind.

Mittels der Drucksensoren sind sowohl die Druckverhältnisse beim Einatmen als auch beim Ausatmen zu bestimmen.

Gassensoren nach dem Stand der Technik weisen die Eigenschaft auf, dass die Gassensoren nach einer erfolgten Messung einen Ausgleichszeitraum benötigen. Die Anordnung der Drucksensoren in dem ersten Gasmessraum 2 erlaubt das Messen des Fluiddrucks während des Einatmens und während des Ausatmens. Das Anordnen der zweiten Gassensoren in dem zweiten Gasmessraum 3 erlaubt das ausschließliche Bestimmen der Bestandteile der ausgeatmeten Luft. Die Gassensoren können sich während des Einatmens ausgleichen.

Der Fachmann wird erfindungsgemäß auch im ersten Gasmessraum 2 erste Gassensoren anordnen, wobei bei einer solchen Anordnung von ersten Gassensoren in dem ersten Gasmessraum 2 der oben beschriebene Vorteil hinsichtlich eines Ausgleiches für die in dem ersten Gasmessraum 2 angeordneten Gassensoren wegfallen würde.

Es sind die Gassensoren und die Drucksensoren in Figur 1 nicht dargestellt.

Mittels der Pumpvorrichtung 6 kann während des Einatmens und/oder während des Ausatmens ein Luftimpuls in den ersten Gasmessraum 2 eingebracht werden. Nachdem der erste Gasmessraum 2 über das Mundstück 1 mit der Lunge des Patienten in Fluidkommunikation steht, kann über die Pumpvorrichtung 6 der Fluiddruck in den Atemwegen des Patienten, insbesondere in der Lunge geändert werden.

Bei der in Figur 1 dargestellten Vorrichtung weist die Pumpvorrichtung 6 ein Verschiebevolumen von circa 40ml und eine Pumpfrequenz von maximal 32 Hertz auf. Diese Parameter sind in der Impuls-Oszillometrie nach der gängigen Lehre üblich. Der Fachmann kann auch andere Parameter wählen und hierdurch besondere Effekte in der Messung und in der Wirkung auf die Eigenschaften der Lunge erzielen, sofern dies durch die gängige Lehre bekannt ist.

Bei der in Figur 1 gezeigten Ausführungsform ist die Pumpvorrichtung 6 in Form eines Membrankompressors ausgebildet. Es wirkt sohin eine in eine Bewegung versetzbare Membran als Kolben 27 der Pumpvorrichtung 6.

Die Membran kann in eine schwingende Bewegung versetzt werden, wodurch ein schwingender Luftimpuls auf den ersten Gasmessraum 2 ausgeübt wird. Die Pumpvorrichtung 6 umfassend eine schwingende Membran als Kolben 27 kann durch einen Lautsprecher ausgebildet sein. Der Lautsprecher oder eine schwingende Membran im Allgemeinen hat den Vorteil, dass nur kleine Massen bewegt werden und sohin keine störenden Vibrationen oder Impulse auf die Vorrichtung ausgeübt werden.

Die Membran kann auch in eine Endlage gebracht werden und in dieser Endlage um eine Zeitspanne gehalten werden, wodurch ein einmaliger Luftimpuls auf den ersten Gasmessraum 2 ausgeübt wird.

Die Vorrichtung umfasst eine Elektronik und elektronische Bauteile wie Mikrocontroller, Analog-DigitalWandler, Verstärkerschaltungen, Prozessoren, Rechner und Speicher. Es kann weiters auch eine Batterie vorgesehen sein, um eine Konstantspannungsversorgung für die empfindliche Auswerteelektronik sicherzustellen.

In vorteilhafter Weise kann auch die Batterie zur Sicherstellung einer Konstantspannungsversorgung für die empfindliche Auswerteelektronik wie beispielsweise den Stickstoffmonoxid-Sensor als Gassensor umfassen. So kann die Stabilisationszeit nach dem Einschalten verkürzt werden, da die mit Konstantspannung versorgten Sensoren dauerhaft eingeschwungen sind.

Die Vorrichtung kann auch Mittel zur Datenübertragung umfassen.

Es ist in dem zweiten Gasmessraum 3 zumindest ein zweiter Gassensor zur Bestimmung der chemischen Bestandteile des in den zweiten Gasmessraum 3 eingebrachten Fluids angeordnet. Bei einer aufrechten und abgeschlossenen Fluidkommunikation zwischen dem Mundstück 1 und dem zweiten Gasmessraum 3 entspricht das in den zweiten Gasmessraum 3 eingebrachte Fluid der aus der Lunge des Patienten ausgebrachten Luft.

Die Vorrichtung umfasst zumindest einen der folgenden Gassensoren: Stickstoffmonoxid-Sensor, Kohlenstoffdioxid-Sensor, Sauerstoff-Sensor, Kohlenmonoxid-Sensor Multi-Gas-Sensor, und/oder Sensoren für flüchtige organische Verbindungen wie Alkane-Sensor, Alkene-Sensor, Aldehyde-Sensor.

Der zweite Gasmessraum 3 umfasst eine verschließbare vierte Öffnung 28 als Gasmessraumauslass. Die vierte Öffnung 28 ist während der Durchführung einer Messung mittels des Gassensors verschlossen, um ein Austreten der zu messenden Atemluft aus dem zweiten Gasmessraum 3 zu unterbinden. Nach einer durchgeführten Messung kann die vierte Öffnung 28 geöffnet werden, um ein Austreten der zuvor in der zweiten Gasmessraum 3 gemessenen Atemluft zu ermöglichen.

Der Fachmann kann in der vierten Öffnung 28 einen Ventilator vorsehen, welcher Ventilator die in der zweiten Gasmessraum 3 enthaltene Luft austreibt.

Die vierte Öffnung 28 kann auch ein weiteres Ventil umfassen, welches weitere Ventil bei der Einbringung von frischer Atemluft in den zweiten Gasmessraum 3 aufgrund des herrschenden Druckes geöffnet ist, um so einen Austausch der in dem zweiten Gasmessraum 3 gehaltenen Luft durch die frische Atemluft zu ermöglichen.

Der zweite Gasmessraum 3 weist bei einer Versuchsvorrichtung ein zweites Gasmessraumvolumen von circa 160,0cm³ auf.

Der Fachmann kann das erste Gasmessraumvolumen in Abhängigkeit des von einem Patienten in die Vorrichtung einbringbaren Luftvolumens abzüglich des Volumens des zweiten Gasmessraumes 3 festlegen. Das erste Gasmessraumvolumen kann kleiner sein als das zweite Gasmessraumvolumen. Dies hat den Vorteil, dass der Patient nach einem Ausatemvorgang und einem Einbringen seiner Atemluft in die Vorrichtung eine größere Menge der Atemluft in den zweiten Gasmessraum 3 einbringt und sohin der Patient nach dem Ausatemvorgang mit einem Großteil seiner Atemluft nicht mehr in Berührung kommt. Dies hat auch zur Folge, dass der Patient den Großteil seiner in die Vorrichtung eingebrachten Atemluft beim Einatmen und sohin beim Ansaugen von Luft durch die Vorrichtung nicht mehr einatmen kann.

Der Fachmann kann sohin das erste Gasmessraumvolumen so groß wählen, dass das beim Einatmen aus dem ersten Gasmessraum 2 gesaugte Luftvolumen keinen Einfluss auf die nachfolgenden Messungen hat und sohin ein Großteil des eingeatmeten Luftvolumens über die Lüftungsschlitze 5 aus der Umgebung entnommen wird.

Der Fachmann kann das erste Gasmessraumvolumen möglichst klein wählen. Bei der bereits oben erwähnten Testvorrichtung weist das erste Gasmessraumvolumen eine Größe von circa 250,0cm³, in einer Modifikation 160,0cm³ auf.

Die Figur 2 zeigt eine Schnittansicht der in Figur 1 dargestellten Ausführungsform der Vorrichtung. Es ist die obige zu Figur 1 abgegebene Beschreibung sinngemäß auf die Figur 2 anzuwenden.

Die in Figur 1 und Figur 2 gezeigte Ausführungsform zeichnet sich dadurch aus, dass der erste Gasmessraum 2 und der zwei Gasmessraum 3 räumlich getrennt sind.

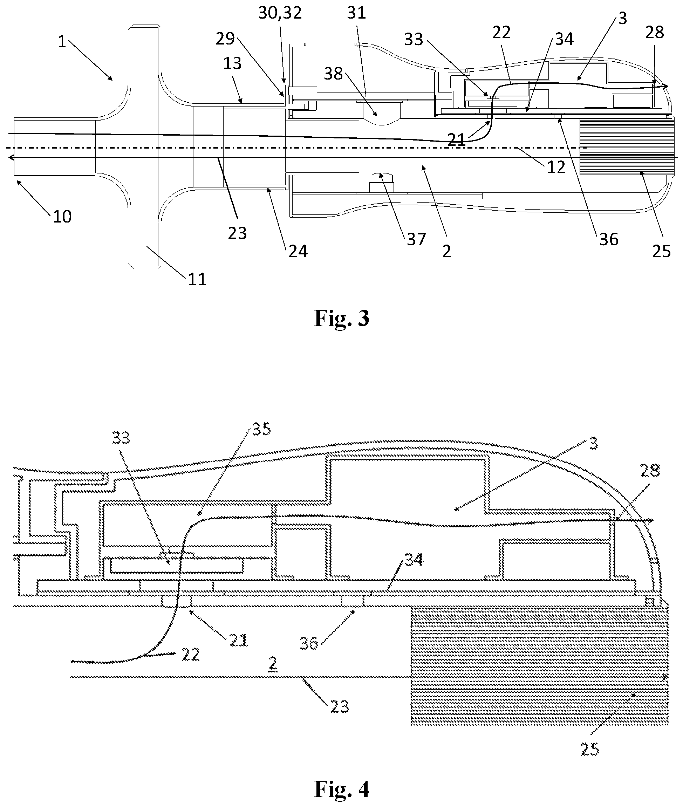

Figur 3 zeigt eine weitere Ausführungsform der Vorrichtung zur Ermittlung von Messwerten beschreibend die Funktion einer Lunge oder des respiratorischen Systems eines Patienten.

Die in Figur 3 gezeigte Vorrichtung umfasst ein Mundstück 1 umfassend ein Rohr zum Einbringen von Atemluft und Ansaugen von Luft. Das Rohr umfasst eine Scheibe 11, welche Scheibe 11 in ihrer flächigen Ausdehnung im Wesentlichen rechtwinkelig zu der Rohrlängsachse angeordnet ist.

Der Patient führt das freie Ende 10 des Mundstückes 1 in seinen Mundraum ein. Die Lippen des Patienten umschließen das freie Ende 10. Auf diese Weise kann der Patient leicht einen dichten Abschluss zwischen dem Mundstück 1 und seinen Lippen herstellen, wodurch gewährleistet ist, dass die Atemluft in das Mundstück 1 und somit in den in Strömungsrichtung gesehen nachgeschalteten Gasmessraum einbringt.

Es kann im Inneren der Scheibe ein Filter angeordnet sein. Die Vorrichtung kann einen Virenfilter und/oder Bakterienfilter und/oder einen Filter zur Entnahme der in der Atemluft enthaltenen Flüssigkeit angeordnet sein. Üblicherweise ist in der Atemluft ein hoher Anteil von Flüssigkeit enthalten; die in der Atemluft enthaltene Flüssigkeit kann jedoch die Vorrichtung beschädigen oder die Qualität der Messung beeinflussen. Aus diesem Grund wird der Atemluft die Feuchtigkeit mittels eines geeigneten Filters im Mundstück 1 entnommen.

Die Vorrichtung umfasst einen Gasmessraum 2, 3, welcher Gasmessraum mit dem Mundstück 1 in Fluidkommunikation steht. Der Patient bringt über das Rohr des Mundstückes 1 in den Gasmessraum 2, 3 Atemluft ein. Vorzugsweise sind der Gasmessraum 2, 3 und das Mundstück 1 entlang einer Erstreckungsachse 12 angeordnet, um keinen Strömungsverlusten unterworfen zu sein und um einen möglichst laminaren Luftfluss auszubilden. Das Mundstück 1 und der Gasmessraum 2, 3 können einen kreisförmigen oder anderen Querschnitt aufweisen, sodass die Teile leicht ineinander steckbar sind.

Das Mundstück 1 weist ein zum freien Ende 10 abgewandtes, angeschlossenes Ende 13 auf, welches angeschlossene Ende 13 luftdicht an der zweiten Öffnung 24 des Gasmessraum 2, 3 angeschlossen ist. Der Anschluss wird beispielsweise über eine lösbare, zu der Umgebung der Vorrichtung dicht abschließende Steckverbindung erreicht. Mittels einer im Bereich der zweiten Öffnung 24 angeordneten Schaltflanke 29 kann überprüft werden, ob das Mundstück gegebenenfalls mitsamt dem Filter korrekt an der zweiten Öffnung 24 des Gasmessraumes 2, 3 angeschlossen ist. Bei einer ausschließlich vorgesehenen Verwendung der Vorrichtung mit einen Filter umfassenden Mundstücken 1 kann über die Schaltflanke 29 überprüft werden, ob ein Mundstück 1 auf den Gasmessraum 2, 3 aufgesetzt ist. Es kann so eine unabsichtliche Verunreinigung der Vorrichtung, insbesondere des Gasmessraumes 2, 3 durch eine Verwendung ohne Mundstück unterbunden werden.

Die Längsachse des Rohres des Mundstückes und der erste Gasmessraum 2 sind entlang einer Erstreckungsachse 12 ausgerichtet.

Die Vorrichtung kann einen Akku zur Versorgung des elektrischen und elektronischen Geräts mit Stromenergie umfassen. Vorzugsweise verschließt das aufgesetzte Mundstück einen Ladestecker 30 zum Laden des Akkus, um so ein Laden des Akkus während des Durchführens von Messungen zu unterbinden. Der Ladestecker 30 ist mit einer Steuerungseinheit verbunden und kann neben dem Zuführen von Strom in den Akku auch als ein Datenkommunikationsmittel 32 dienen. Über das Datenkommunikationsmittel 32 können Daten wie beispielsweise Messwerte auf die Steuerungseinheit 31 gespeichert oder von dieser geladen werden. Weiters kann die auf der Steuerungseinheit 31 abgespeicherte Software verändert werden. Es ist in dem Gasmessraum 2, 3 zumindest einer der folgenden Gassensoren zur Bestimmung der relevanten Messwerte angeordnet:
Stickstoffmonoxid-Sensor, Kohlenstoffdioxid-Sensor, Sauerstoff-Sensor, Kohlenmonoxid-Sensor, Multi-Gas-Sensor, Sensor für flüchtige organische Verbindungen (wie Alkane-Sensor, Alkene-Sensor, Aldehyde-Sensor), Alkane-Sensor, Infrarotsensor und/oder Lichtwellensensor und/oder Widerstandssensor und/oder Halbleitersensor. Erfindungsgemäß fallen nur Ausführungsbeispiele unter die Ansprüche, wenn in dem zweiten Gasmessraum einer der folgenden Gassensoren zur Bestimmung von relevanten Messwerten angeordnet ist: Stickstoffmonoxid-Sensor, Kohlenstoffdioxid-Sensor, Sauerstoff-Sensor, Kohlenmonoxid-Sensor, Multi-Gas-Sensor, Sensor für flüchtige organische Verbindungen, Alkane-Sensor, Alkene-Sensor, oder Aldehyde- Sensor. Erfindungsgemäß fallen nur Ausführungsbeispiele unter die Ansprüche, wenn in dem ersten Gasmessraum einer der folgenden Gassensoren zur Bestimmung von relevanten Messwerten angeordnet ist: Kohlenstoffdioxid-Sensor, Kohlenmonoxid-Sensor.

Die Vorrichtung zeichnet sich dadurch aus, dass der Gasmessraum in einen ersten Gasmessraum 2 und in einen zweiten Gasmessraum 3 durch eine verschließbare Öffnung räumlich getrennt ist. Die verschließbare Öffnung gibt beim Ausatmen und somit beim Einbringen von Atemluft in die Vorrichtung einen ersten Strömungspfad 22 von dem ersten Gasmessraum 2 in den zweiten Gasmessraum 3 frei.

Die räumliche Trennung zwischen dem ersten Gasmessraum 2 und dem zweiten Gasmessraum 3 ist derart, dass das in dem zweiten Gasmessraum 3 eingeschlossene Luftvolumen von dem ersten Gasmessraum 2 und von der Umgebung der Vorrichtung trennbar ist.

In dem zweiten Gasmessraum 3 ist zumindest ein zweiter Gassensor angeordnet. Die in den zweiten Gasmessraum 3 eingebrachte Atemluft kann über eine vierte Öffnung 28 abgegeben werden. Im Sinne der oben erwähnten Trennung des im zweiten Gasmessraum 3 eingeschlossenen Luftvolumens ist die vierte Öffnung als ein Ventil oder als ein Rückschlagventil ausgebildet.

Bei der in Figur 3 gezeigten Ausführungsform der Vorrichtung ist die verschließbare Öffnung als eine Gasmessraum-Pumpvorrichtung 33 ausgebildet. Durch die Positionierung des Kolbens der Gasmessraum-Pumpvorrichtung in einer eine Fluidkommunikation zwischen dem ersten Gasmessraum 2 und dem zweiten Gasmessraum 3 gestattenden Stellung kann die verschließbare Öffnung geöffnet werden. Durch die Positionierung des Kolbens in einer eine solche Fluidkommunikation unterbindenden Stellung kann die verschließbare Öffnung geschlossen werden. Der Fachmann kann zur Positionierung des Kolbens eine Steuerung nach dem Stand der Technik einsetzen. Weiters ist es möglich, mittels der Gasmessraum-Pumpvorrichtung 33 eine definierte Menge von dem ersten Gasmessraum 2 in den zweiten Gasmessraum 3 zu bringen.

Die erste Öffnung 21 ist nicht an der Erstreckungsachse 12 angeordnet, sodass beim Einbringen der Atemluft von dem ersten Gasmessraum 2 in den zweiten Gasmessraum 3 Strömungsverluste auftreten, welche Strömungsverluste durch die Saugwirkung der Gasmessraum-Pumpvorrichtung 33 ausgeglichen werden können.

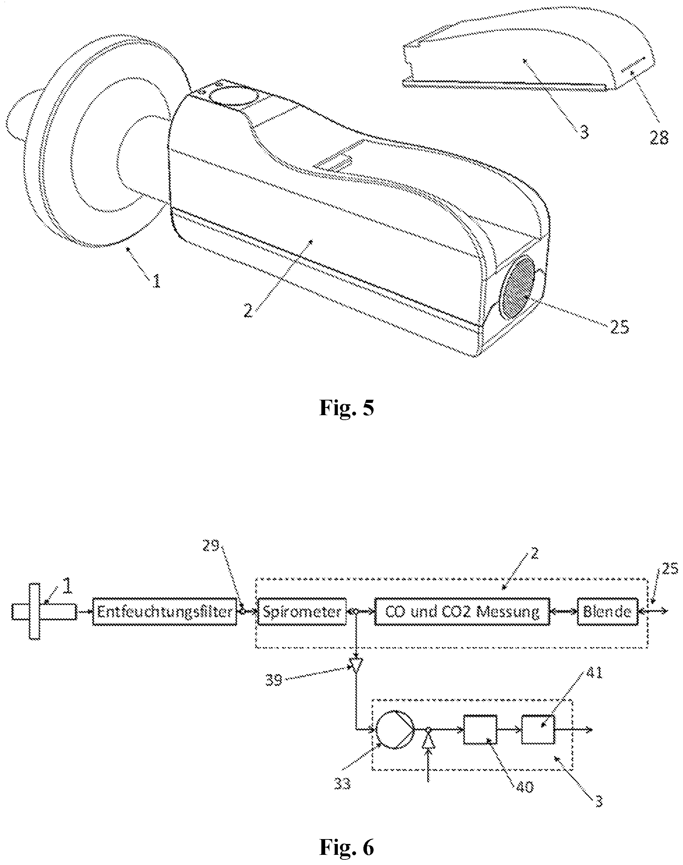

Die Figur 4 zeigt eine Detailansicht der Vorrichtung. Es erstreckt sich zwischen dem ersten Gasmessraum 2 und dem zweiten Gasmessraum 3 eine dichte Schicht, welche dichte Schicht lediglich an definierten Stellen unterbrochen ist. Die dichte Schicht kann beispielsweise als die elastische Dichtung 34 ausgebildet sein. Der zweite Gasmessraum 3 kann mitsamt der Dichtung von dem ersten Gasmessraum 2 unter Lösen einer mechanischen Verbindung wie beispielsweise einer Rastverbindung abnehmbar ausgeführt sein, wie dies in Figur 5 dargestellt ist. In vorteilhafter Weise kann die Dichtung 34 an der Außenoberfläche des zweiten Gasmessraumes 3 haftend ausgeführt sein, sodass bei einem Austausch des zweiten Gasmessraumes 3 auch die Dichtung 34 ausgetauscht wird.

Bei der in Figur 3 dargestellten Ausführungsform wird die Atemluft mittels der Gasmessraum-Pumpenvorrichtung 33 vom ersten Gasmessraum 2 in den zweiten Gasmessraum 3 gepumpt. Die Atemluft folgt hierbei dem ersten Strömungspfad 22. Die zweiten Gassensoren sind im zweiten Gasmessraum 3 angeordnet, wodurch die im Rahmen der Offenbarung der Vorrichtung vorteilhaften Effekte erzielt werden können. Die Gasmessraum-Pumpvorrichtung 33 wirkt als eine geöffnete Öffnung zwischen dem ersten Gasmessraum 2 und dem zweiten Gasmessraum 3, sodass die Atemluft dem ersten Strömungspfad 22 folgend von dem ersten Gasmessraum 2 in den zweiten Gasmessraum 3 strömen kann.

Die Gasmessraum-Pumpvorrichtung 33 kann auch als eine verschlossene Öffnung zwischen dem ersten Gasmessraum 2 und dem zweiten Gasmessraum 3 wirken, sodass die Atemluft dem zweiten Strömungspfad 23 folgend strömt.

Die in dem zweiten Gasmessraum 3 angeordneten zweiten Gassensoren messen hierbei unter Anwendung der gängigen Lehre in Abhängigkeit der Eigenschaft der zweiten Gassensoren die Konzentration bestimmter Gase.

Die Vorrichtung, insbesondere der zweite Gasmessraum 3 ist wegen seiner räumlichen Trennung vom ersten Gasmessraum 2 in seiner Form an die jeweiligen zweiten Gassensoren anpassbar, um unter Berücksichtigung der Eigenschaften der angeordneten zweiten Gassensoren ein optimales Umströmen und somit Beaufschlagen der jeweiligen zweiten Gassensoren zu erreichen. Der Fachmann kann die optimale Form des zweiten Gasmessraumes 3 beispielsweise durch Simulationen nach der gängigen Lehre bestimmen.

Die dem ersten Strömungspfad 22 folgende Atemluft kann hierbei einen zweiten Gasmessteilraum 35 passieren, in welchem Gasmessteilraum 35 ein Katalysator und/oder Oxidator und/oder einer Reagens nach dem Stand der Technik zur Oxidation des Stickstoffmonoxids zu Stickstoffdioxid angeordnet ist. In der Atemluft von Patienten mit bekannten Erkrankungen ist Stickstoffmonoxid enthalten. Der zweite Gasmessteilraum 35 kann eine stets geöffnete Öffnung zu dem zweiten Gasmessraum 3 umfassen.

Der zweite Gasmessraum 3 kann Teilbereiche umfassen, in welchen Teilbereichen Steuerungselemente angeordnet sind. Vorzugsweise werden Steuerungselemente so angeordnet, dass diese von der Atemluft stets getrennt sind. Alternativ zu der Ausbildung von Teilbereichen können die Steuerungselemente auch mit einer Dichtlackschicht versehen werden.

Die in Figur 3 gezeigte Vorrichtung kann in Ergänzung zu der Messung mittels der in dem zweiten Gasmessraum 3 angeordneten zweiten Gassensoren sind auch weitere Sensoren zur Durchführung weiterer Messungen erfordern. Der Fachmann ist insbesondere in der Lage, die oben beschriebene Anordnung der zweiten Gassensoren mit anderen Sensoren der obigen Beschreibung ergänzen oder auszutauschen.

Der Fachmann kann beispielsweise im Bereich der zweiten Öffnung 24 des Gasmessraumes 2, 3 erste Gasmesssensoren zur Messung der Gaskonzentration von Kohlendioxid und/oder Kohlenmonoxid anordnen. Der Fachmann kann hierbei erste Gassensoren anordnen, welche erste Gassensoren eine Messung mittels NDIR Verfahren (nondispersive infrared Verfahren mit einem ersten Emitter 37 und einem ersten Detektor 38) erlauben. Der Fachmann ordnet hierzu einen Emitter und einen Detektor an gegenüberliegenden Innenoberflächen des ersten Gasmessraumes 2 an. Der Fachmann bildet den ersten Gasmessraum 2 beispielsweise mit einem runden Querschnitt aus. Allenfalls wählt der Fachmann die Dimensionen des Querschnittes so, dass der Emitter und der Detektor in einem zur Durchführung der Messung optimalen Abstand voneinander entfernt an der Innenoberfläche des ersten Gasmessraumes 2 anordenbar sind.

Der erste Gasmessraum 2 umfasst neben der ersten Öffnung 21, welche erste Öffnung 21 die Verbindung zu dem zweiten Gasmessraum 3 darstellt, und der zweiten Öffnung 24, an welcher zweiten Öffnung 24 das Mundstück 1 angeschlossen ist, eine dritte Öffnung 25, durch welche dritte Öffnung 25 die nicht in den zweiten Gasmessraum 3 eingebrachte Atemluft an die Umgebung abgegeben wird. Im Wesentlichen wird der Großteil der Atemluft über die dritte Öffnung 25 abgegeben. Der im ersten Gasmessraum 2 herrschende Druck und/oder der im ersten Gasmessraum 2 herrschende Durchfluss kann mittels eines ersten Drucksensors 36 beziehungsweise eines Durchflusssensors ermittelt werden, welcher Drucksensor 36 beziehungsweise Durchflusssensor in der Richtung des zweiten Strömungspfades 23 gesehen nach der ersten Öffnung 21 angeordnet ist.

Bei der in Figur 3 dargestellten Ausführungsform umfasst die dritte Öffnung 25 eine Vielzahl von Röhrchen zur Ausbildung einer im Wesentlichen laminaren Strömung. Diese laminare Strömung erlaubt in einer vorteilhaften Weise die Bestimmung des im ersten Gasmessraum 2 herrschenden Druckes mittels des ersten Drucksensors 36, da dadurch eine annähernd homogene Druckverteilung im Gasmessraum herrscht. Ebenso ist in vorteilhafter Weise der Durchfluss mittels des Durchflusssensors bestimmbar. Es ist in der Figur 3 lediglich der erste Drucksensor 36 eingetragen; der Fachmann kann an der Stelle des ersten Drucksensors 36 den ersten Durchflusssensor anordnen.

Die Anordnung von Röhrchen in der dritten Öffnung ist - wie oben dargestellt - in Hinblick auf die Druckmessung in der dritten Öffnung 25 vorteilhaft, jedoch nicht zwingend. Durch diese Anordnung der Röhrchen in der dritten Öffnung 25 wird auch bewerkstelligt, dass bei einer geöffneten Öffnung die eingebrachte Atemluft dem zweiten Strömungspfad 22 folgt, was - wie oben dargestellt - auch durch die Gasmessraum-Pumpvorrichtung 33 unterstützt wird.

Der Fachmann ordnet weiters einen Drucksensor zur Bestimmung des Umgebungsdruckes an.

Figur 5 zeigt eine dreidimensionale Darstellung der Vorrichtung. Es wird in Figur 5 die Abnehmbarkeit des zweiten Gasmessraumes 3 vom ersten Gasmessraum 2 beziehungsweise jener strukturellen Teile, welche Teile die jeweiligen Gasmessräume 2, 3 ausbilden, veranschaulicht. Wie oben erläutert ist es vorteilhaft, den zweiten Gasmessraum 3 bei einer definierten Anzahl von Messungen auszutauschen. Die Anzahl der Messungen kann durch ein Zählwerk angezeigt werden.

Es ist weiters das Mundstück 1 von dem ersten Gasmessraum 2 abnehmbar gestaltet. Der Fachmann sieht hier eine einfache Steckverbindung vor.

Die Austauschbarkeit des Mundstückes 1 und/oder des zweiten Gasmessraumes 3 sind Maßnahmen zur Dekontamination, welche Maßnahmen der Fachmann unter Beachtung von Richtlinien durchführen kann. Die in Figur 3 gezeigte Ausführungsform ist als ein Prototyp umgesetzt.

Der Prototyp umfasst ein austauschbares Mundstück vom Typ CareFusion - MicroGard^{®} IIB Bakterien/Viren-Filter.

Bei dem Prototyp wird ein optischer Näherungssensor als zur Detektion eines auf den Gasmessraum aufgebrachten Mundstückes verwendet. Es wird der Sensor VCNL4040 des Anbieters Vishay Semicondctors als Flankenschalter 29 eingesetzt.

Bei dem Prototyp wird als erster Emitter 37 ein Infrarot Strahler oder Infrarot Emitter wie beispielsweise der Emitter HSL-EMIRS200_R_60/55_0 des Anbieters Heimann eingesetzt. Dieser Emitter zeichnet sich durch die Abstrahlung eines breitbandigen und leistungsstarken Spektrums im Infrarotbereich aus.

Es wird als erster Detektor 38 beispielsweise HTS Multichannel Sensor des Anbieters Heimann oder ein Sensor LRM-244-HDEI-12 des Anbieters InfraTec eingesetzt. Wie oben ausgeführt kann mittels des NDIR-Verfahrens, zu welchem Verfahren diese Sensoren eingesetzt werden, unter gegebenenfalls Verwendung von Filtern im Mundstück 1 die Konzentration von Kohlenstoffmonoxid und/oder Kohlenstoffdioxid gemessen werden.

Die Steuerungseinheit 31 des Prototyps ist vorzugsweise so ausgebildet, dass unterschiedliche erste Emitter 37 und/oder erste Detektoren 38 einsetzbar sind.

Der erwähnte Prototyp umfasst als Gasmessraum-Pumpvorrichtung eine Pumpe vom Typ MZB1001T02 des Anbieters Murata.

In dem zweiten Gasmessraum 3 ist ein Sensor zur Bestimmung der Temperatur und/oder Feuchtigkeit und/oder des Druckes angeordnet. Es wird ein Sensor vom Type BME280 des Anbieters Bosch eingesetzt. Wie oben ausgeführt, ist dieser Sensor optional; der Einsatz dieses Messsensors kann die Qualität der Messungen erhöhen.

Im zweiten Gasmessraum 3 ist der zweite Gassensor angeordnet, um die oben erwähnten vorteilhaften Effekte zu erreichen. Im Falle des Prototyps ist ein Sensor vom Typ 4OX des Anbieters SGX Sensortech und/oder ein beliebiger SPEC Sensor im zweiten Gasmessraum 3 anordenbar. In einer vorteilhaften Weise ist die Vorrichtung so ausgebildet, dass unterschiedliche Sensoren und/oder mehrere Sensoren in dem zweiten Gasmessraum 3 anordenbar sind.

Es ist im zweiten Gasmessraum 3 zusätzlich oder alternativ zu den erwähnten Sensoren ein Sensor der Type MICS-2714 des Anbieters SGX Sensortech anordenbar.

Wie oben erläutert ist die Messung des Druckes und/oder der Temperatur und/oder der Feuchtigkeit im ersten Gasmessraum 2 vorteilhaft. Dies kann erforderlich sein, um relevante Messwerte aus der Spirometrie zu erfassen. Der gemessene Feuchtigkeitswert in der Atemluft kann als ein Indiz für die Qualität der mit der Vorrichtung durchgeführten Messung sein und gegebenenfalls als ein Fehlerindikator angesehen werden. Bei dem Prototyp wird ein Sensor vom Typ BME280 des Anbieters Bosch eingesetzt.

In vorteilhafter Weise werden auch relevante Werte der Umgebung wie Temperatur und/oder Feuchtigkeit und/oder Druck gemessen. Im Falle des Prototyps erfolgt diese Messung mit Hilfe des Sensors vom Typ BME280 oder BMP280 des Anbieters Bosch.

Die Vorrichtung kann ein Datenkommunikationsmittel wie beispielsweise eine Funkverbindung zur Übertragung der ermittelten Daten an ein Datenverarbeitungsgerät umfassen. Das Datenverarbeitungsgerät kann ein handelsüblicher Computer sein.

Die Figur 6 veranschaulicht in einem Blockdiagramm die einzelnen Komponenten eines weiteren Prototypen. Ergänzend zu dem in Figur 3 bis Figur 5 dargestellten Prototyp umfasst der weitere Prototyp an den eingetragenen Positionen Rückschlagventile 39. Der sonstige Aufbau des weiteren Prototyps entspricht im Wesentlichen dem Prototyp.

## Patentansprüche

1. Vorrichtung zur Ermittlung von Messwerten beschreibend die Funktion der Lunge oder des respiratorischen Systems eines Patienten umfassend als Vorrichtungselemente
ein Mundstück (1) umfassend ein Rohr zum Einbringen von Atemluft und Ansaugen von Luft, einen ersten Gasmessraum (2),
einen zweiten Gasmessraum (3), wobei der erste Gasmessraum (2) und der zweite Gasmessraum (3) durch eine verschließbare erste Öffnung (21) getrennt sind, wobei die verschließbare erste Öffnung (21) eine Gasmessraum-Pumpvorrichtung (33) und/oder ein Ventil (4, 39) umfasst, welche verschließbare erste Öffnung (21) beim Ausatmen und sohin Einbringen von Atemluft in die Vorrichtung einen ersten Strömungspfad (22) freigibt, welcher von der ersten Öffnung (21) des ersten Gasmessraums (2) in den zweiten Gasmessraum (3) führt,
wobei der erste Gasmessraum (2) neben der ersten Öffnung (21), welche erste Öffnung (21) die Verbindung zu dem zweiten Gasmessraum (3) darstellt und einer zweiten Öffnung (24), an welcher zweiten Öffnung (24) das Mundstück (1) angeschlossen ist, eine dritte Öffnung (25) umfasst, durch welche dritte Öffnung (25) die nicht in den zweiten Gasmessraum (3) eingebrachte Atemluft über einen zweiten Strömungspfad (23) an die Umgebung abgegeben wird, wobei bei verschlossener erster Öffnung (21) zwischen dem ersten Gasmessraum (2) und dem zweiten Gasmessraum (3) die Atemluft dem zweiten Strömungspfad (23) folgend strömt,
wobei das Mundstück (1) und der erste Gasmessraum (2) in einer Fluidkommunikation zueinander stehen,
wobei in dem zweiten Gasmessraum (3) einer der folgenden Gassensoren zur Bestimmung von relevanten Messwerten angeordnet ist: Stickstoffmonoxid-Sensor, Kohlenstoffdioxid-Sensor, Sauerstoff-Sensor, Kohlenmonoxid-Sensor, Multi-Gas-Sensor, Sensor für flüchtige organische Verbindungen, Alkane-Sensor, Alkene-Sensor oder Aldehyde-Sensor,
wobei
auch im ersten Gasmessraum (2) einer der folgenden Gassensoren zur Bestimmung von relevanten Messwerten angeordnet ist: Kohlenstoffdioxid-Sensor, Kohlenmonoxid-Sensor,
wobei der im ersten Gasmessraum (2) angeordnete Gassensor ein Kohlenstoffdioxid-Sensor oder Kohlenmonoxid-Sensor ist,
wobei der zweite Gasmessraum (3) vom ersten Gasmessraum (2) abnehmbar ist und
wobei sich zwischen dem ersten Gasmessraum (2) und dem zweiten Gasmessraum (3) eine dichte Schicht befindet, welche dichte Schicht lediglich an definierten Stellen unterbrochen ist, wobei die dichte Schicht als eine elastische Dichtung (34) ausgebildet ist, wobei die Dichtung (34) an der Außenoberfläche des zweiten Gasmessraumes (3) haftend ausgeführt ist, sodass bei einem Austausch des zweiten Gasmessraumes (3) auch die Dichtung (34) ausgetauscht wird.

2. Vorrichtung nach einem der Ansprüche 1 bis 2, **dadurch gekennzeichnet, dass** der im ersten Gasmessraum (2) im Bereich der zweiten Öffnung (24) angeordnete Gassensor einen Emitter (37) und einen Detektor (38) umfasst, welche an gegenüberliegenden Innenoberflächen des ersten Gasmessraumes (2) vorliegen, zur Messung der Gaskonzentration von Kohlendioxid und/oder Kohlenmonoxid mittels NDIR Verfahren, also einem Nondispersive-infrared-Verfahren.

3. Vorrichtung nach einem der Ansprüche 1 bis 2, **dadurch gekennzeichnet, dass** die verschließbare Öffnung die als Alternative genannte Gasmessraum-Pumpvorrichtung (33) umfasst, welche Gasmessraum-Pumpvorrichtung (33) als eine geöffnete Öffnung zwischen dem ersten Gasmessraum (2) und dem zweiten Gasmessraum (3) wirkt, indem die Gasmessraum-Pumpvorrichtung (33) die Atemluft mittels der Gasmessraum-Pumpenvorrichtung (33) vom ersten Gasmessraum (2) in den zweiten Gasmessraum (3) pumpt.

4. Vorrichtung nach einem der Ansprüche 1 bis 3, **dadurch gekennzeichnet, dass** der zweite Gasmessraum (3) zudem einen Gasmessteilraum (35) umfasst, in welchem Gasmessteilraum (35) ein Katalysator und/oder Oxidator und/oder ein Reagens zur Oxidation von Stickstoffmonoxid zu Stickstoffdioxid angeordnet ist.

5. Vorrichtung nach einem der Ansprüche 1 bis 4, **dadurch gekennzeichnet, dass** der zweite Gasmessraum (3) zumindest einen Teilbereich umfasst, in welchem Steuerungselemente angeordnet sind.

6. Vorrichtung nach Anspruch 5, **dadurch gekennzeichnet, dass** der zweite Gasmessraum (3) vom ersten Gasmessraum (2) unter Lösen einer mechanischen Verbindung, wie beispielsweise einer Rastverbindung, abnehmbar ausgeführt ist.

7. Vorrichtung nach einem der Ansprüche 1 bis 6, **dadurch gekennzeichnet, dass** die Längsachse des Rohres des Mundstückes (1) und der erste Gasmessraum (2) entlang einer Erstreckungsachse (12) ausgerichtet sind.

8. Vorrichtung nach einem der Ansprüche 1 bis 7, **dadurch gekennzeichnet, dass**
im Mundstück (1) ein weiterer Gassensor angeordnet ist.

9. Vorrichtung nach einem der Ansprüche 1 bis 8, **dadurch gekennzeichnet, dass**
der Gassensor im zweiten Gasmessraum (3) als Widerstandssensor oder Halbleitersensor ausgebildet ist und einen Trägerkörper mit einer Trägerkörperoberfläche aufweist,
auf welcher Trägerkörperfläche isolierte Leiterbahnen mit einer Leiterbahnoberfläche angeordnet sind,
auf welcher Leiterbahnoberfläche ein Messkörper umfassend Tetracosane und ein Bindemittel umfassend Acrylic Copolymer, Polyurethane Polymer angeordnet ist.

10. Vorrichtung nach einem der Ansprüche 1 bis 9, **dadurch gekennzeichnet, dass** der im ersten Gasmessraum (2) herrschende Druck und/oder der im ersten Gasmessraum (2) herrschende Durchfluss mittels eines ersten Drucksensors (36) beziehungsweise eines Durchflusssensors ermittelt wird, welcher Drucksensor (36) beziehungsweise Durchflusssensor in der Richtung des zweiten Strömungspfades (23) gesehen nach der ersten Öffnung (21) angeordnet ist.

11. Vorrichtung nach einem der Ansprüche 1 bis 10, **dadurch gekennzeichnet, dass**
die Vorrichtung eine Pumpvorrichtung (6) umfasst, welche Pumpvorrichtung (6) zum Einbringen eines Luftimpulsvolumens in den ersten Gasmessraum (2) mit dem ersten Gasmessraum (2) in Fluidkommunikation steht.

12. Vorrichtung nach einem der Ansprüche 1 bis 11, **dadurch gekennzeichnet, dass**
das Mundstück (1) einen Virenfilter und/oder einen Luftentfeuchter umfasst.

13. Vorrichtung nach einem der Ansprüche 1 bis 12, **dadurch gekennzeichnet, dass**
die Vorrichtung einen in dem ersten Gasmessraum (2) angeordneten ersten Temperatursensor und einen in dem zweiten Gasmessraum (3) angeordneten zweiten Temperatursensor umfasst.

14. Vorrichtung nach einem der Ansprüche 1 bis 13, **dadurch gekennzeichnet, dass**
die Vorrichtung einen in dem ersten Gasmessraum (2) angeordneten ersten Feuchtesensor und einen in dem zweiten Gasmessraum (3) angeordneten zweiten Feuchtesensor umfasst.

15. Vorrichtung nach einem der Ansprüche 1 bis 14, **dadurch gekennzeichnet, dass**
die Vorrichtung
- einen Drucksensor zur Bestimmung des atmosphärischen Umgebungsdruckes der Umgebung der Vorrichtung und/oder
- einen Gassensor zur Bestimmung der Gaszusammensetzung des Umgebungsgases der Umgebung der Vorrichtung und/oder
- einen Temperatursensor zur Bestimmung der Umgebungstemperatur der Umgebung der Vorrichtung und/oder
- einen Feuchtigkeitssensor zur Bestimmung der Umgebungsfeuchte der Umgebung der Vorrichtung umfasst.

16. Vorrichtung nach einem der Ansprüche 1 bis 15, **dadurch gekennzeichnet, dass**
in einem Gasmessraumeinlass (5) eine Heizvorrichtung und/oder eine Kühlvorrichtung zum Heizen beziehungsweise Kühlen der in den Gasmessraum (2, 3) eingebrachten Luft und/oder der Sensoren angeordnet ist.

17. Vorrichtung nach einem der Ansprüche 1 bis 16, **dadurch gekennzeichnet, dass**
die Vorrichtung ein Datenkommunikationsmittel zur Übertragung der mittels des Sensors ermittelten Daten an ein Datenverarbeitungsgerät umfasst.

18. Vorrichtung nach einem der Ansprüche 1 bis 17, **dadurch gekennzeichnet, dass**
die Vorrichtung ein Zählwerk umfasst, welches die Anzahl der Messungen zählt, wobei der zweiten Gasmessraum (3) bei einer definierten Anzahl von Messungen auszutauschen ist.

## Claims

1. A device for determining measurement values describing the functioning of the lung or respiratory system of a patient, comprising the device elements of
a mouthpiece (1) comprising a pipe for introducing respiratory air and taking in air,
a first gas measurement space (2),
a second gas measurement space (3), the first gas measurement space (2) and the second gas measurement space (3) being separated by a sealable first opening (21), wherein the sealable first opening (21) comprises a gas measurement space pumping device (33) and/or a valve (4, 39), which sealable first opening (21), while respiratory air is introduced into the device on the exhale, releases a first flow path (22), which leads from the first opening (21) of the first gas measurement space (2) into the second gas measurement space (3),
wherein the first gas measurement space (2) comprises, in addition to the first opening (21), which first opening (21) constitutes the connection to the second gas measurement space (3), and a second opening (24), to which second opening (24) the mouthpiece (1) is attached, a third opening (25), through which third opening (25) the respiratory air not introduced into the second gas measurement space (3) is dissipated into the environment via a second flow path (23), wherein, when the first opening (21) is sealed, the respiratory air flows between the first gas measurement space (2) and the second gas measurement space (3) following the second flow path (23), wherein the mouthpiece (1) and the first gas measurement space (2) are in fluid communication with one another,
wherein one of the following gas sensors is arranged in the second gas measurement space (3) for determining relevant measurement values: nitric oxide sensor, carbon dioxide sensor, oxygen sensor, carbon monoxide sensor, multi-gas sensor, volatile organic compounds sensor, alkanes sensor, alkenes sensor or aldehyde sensor,
wherein
one of the following gas sensors is also arranged in the first gas measurement space (2) for determining relevant measurement values: carbon dioxide sensor, carbon monoxide sensor,
wherein the gas sensor arranged in the first gas measurement space (2) is a carbon dioxide sensor or a carbon monoxide sensor,
wherein the second gas measurement space (3) is removable from the first gas measurement space (2) and
wherein a dense layer is located between the first gas measurement space (2) and the second gas measurement space (3), which dense layer is merely interrupted in defined positions, wherein the dense layer is formed as an elastic gasket (34), wherein the gasket (34) is made to adhere to the exterior surface of the second gas measurement space (3) such that, when the second gas measurement space (3) is replaced, the gasket (34) is also replaced.

2. The device of claim 1, **characterised in that** the gas sensor arranged in the first gas measurement space (2) in the area of the second opening (24) comprises an emitter (37) and a detector (38), which are present on opposite interior surfaces of the first gas measurement space (2), to measure the gas concentration of carbon dioxide and/or carbon monoxide using an NDIR, i.e. nondispersive infrared, method.

3. The device of any one of claims 1 and 2, **characterised in that** the sealable opening comprises the gas measurement space pumping device (33) mentioned as an alternative, which gas measurement space pumping device (33) acts as an opened opening between the first gas measurement space (2) and the second gas measurement space (3) **in that** the gas measurement space pumping device (33) pumps the respiratory air from the first gas measurement space (2) into the second gas measurement space (3) using the gas measurement space pumping device (33).

4. The device of any one of claims 1 to 3, **characterised in that** the second gas measurement space (3) also comprises a gas measurement subspace (35), in which gas measurement subspace (35) a catalyst and/or oxidiser and/or reagent for oxidising nitric oxide to nitric dioxide is arranged.

5. The device of any one of claims 1 to 4, **characterised in that** the second gas measurement space (3) comprises at least one subspace in which control elements are arranged.

6. The device of claim 5, **characterised in that** the second gas measurement space (3) is made to be removable from the first gas measurement space (2) by releasing a mechanical connection such as a latching connection, for example.

7. The device of any one of claims 1 to 6, **characterised in that** the longitudinal axis of the pipe of the mouthpiece (1) and the first gas measurement space (2) are oriented along an extension axis (12).

8. The device of any one of claims 1 to 7, **characterised in that**
another gas sensor is arranged in the mouthpiece (1).

9. The device of any one of claims 1 to 8, **characterised in that**
the gas sensor in the second gas measurement space (3) is formed as a resistance sensor or semiconductor sensor and has a support body with a support body surface,
on which support body surface isolated conducting paths with a conducting path surface are arranged,
on which conducting path surface a measurement body comprising tetracosanes and a binder comprising acrylic copolymer, polyurethane polymer is arranged.

10. The device of any one of claims 1 to 9, **characterised in that** the pressure in the first gas measurement space (2) and/or the flow in the first gas measurement space (2) is/are determined using a first pressure sensor (36) and a flow sensor, respectively, which pressure sensor (36) or flow sensor is arranged downstream of the first opening (21) as seen in the direction of the second flow path (23).

11. The device of any one of claims 1 to 10, **characterised in that**
the device comprises a pumping device (6), which pumping device (6) is in fluid communication with the first gas measurement space (2) for introducing an air impulse volume into the first gas measurement space (2).

12. The device of any one of claims 1 to 11, **characterised in that**
the mouthpiece (1) comprises a virus filter and/or a dehumidifier.

13. The device of any one of claims 1 to 12, **characterised in that**
the device comprises a first temperature sensor arranged in the first gas measurement space (2) and a second temperature sensor arranged in the second gas measurement space (3).

14. The device of any one of claims 1 to 13, **characterised in that**
the device comprises a first humidity sensor arranged in the first gas measurement space (2) and a second humidity sensor arranged in the second gas measurement space (3).

15. The device of any one of claims 1 to 14, **characterised in that**
the device comprises
- a pressure sensor for determining the barometric ambient pressure of the device's environment and/or
- a gas sensor for determining the gas composition of the ambient gas of the device's environment and/or
- a temperature sensor for determining the ambient temperature of the device's environment and/or
- a humidity sensor for determining the ambient humidity of the device's environment.

16. The device of any one of claims 1 to 15, **characterised in that**
a heating device and/or a cooling device for heating or cooling, respectively, the air introduced into the gas measurement space (2, 3) and/or the sensors is arranged in a gas space inlet (5).

17. The device of any one of claims 1 to 16, **characterised in that**
the device comprises data communication means for transferring the data determined using the sensor to data processing equipment.

18. The device of any one of claims 1 to 17, **characterised in that**
the device comprises a counter which counts the number of measurements, wherein the second measurement space (3) must be replaced at a defined number of measurements.

## Revendications

1. Dispositif pour déterminer des valeurs de mesure décrivant la fonction des poumons ou du système respiratoire d'un patient, comprenant comme éléments du dispositif
un embout buccal (1) comprenant un tube pour l'introduction d'air respiratoire et l'aspiration d'air, une première chambre de mesure des gaz (2), une deuxième chambre de mesure de gaz (3), la première chambre de mesure de gaz (2) et la deuxième chambre de mesure de gaz (3) étant séparées par une première ouverture obturable (21), la première ouverture obturable (21) comprenant un dispositif de pompage de chambre de mesure de gaz (33) et/ou une soupape (4, 39), ladite première ouverture obturable (21), lors de l'expiration et donc de l'introduction d'air respiratoire dans le dispositif , ouvrant un première voie d'écoulement (22), qui mène de la première ouverture (21) du premier espace de mesure de gaz (2) dans la deuxième chambre de mesure de gaz (3), la première chambre de mesure de gaz (2) comprenant, outre la première ouverture (21), ladite première ouverture (21) représentant la liaison avec la deuxième chambre de mesure de gaz (3), et une deuxième ouverture (24), à ladite deuxième ouverture (24) l'embout buccal (1) étant raccordé, une troisième ouverture (25), à travers ladite troisième ouverture (25) l'air expiré qui n'est pas introduit dans la deuxième chambre de mesure de gaz (3) étant délivré à l'environnement par une deuxième voie d'écoulement (23), l'air expiré s'écoulant en suivant la deuxième voie d'écoulement (23) lorsque la première ouverture (21) entre la première chambre de mesure de gaz (2) et la deuxième chambre de mesure de gaz (3) est fermée,
l'embout buccal (1) et la première chambre de mesure de gaz (2) étant en communication fluidique l'un avec l'autre,
l'un des capteurs de gaz suivants étant disposé dans la deuxième chambre de mesure de gaz (3) pour déterminer des valeurs de mesure pertinentes : capteur de monoxyde d'azote, capteur de dioxyde de carbone, capteur d'oxygène, capteur de monoxyde de carbone, capteur multi-gaz, capteur de composés organiques volatils, capteur d'alcanes, capteur d'alcènes ou capteur d'aldéhydes,
l'un des capteurs de gaz suivants étant disposé aussi dans la première chambre de mesure de gaz (2) pour déterminer des valeurs de mesure pertinentes : capteur de dioxyde de carbone, capteur de monoxyde de carbone,
le capteur de gaz disposé dans la première chambre de mesure de gaz (2) étant un capteur de dioxyde de carbone ou un capteur de monoxyde de carbone,
la deuxième chambre de mesure de gaz (3) pouvant être retiré de la première chambre de mesure de gaz (2) et
une couche étanche se trouvant entre la première chambre de mesure de gaz (2) et la deuxième chambre de mesure de gaz (3), ladite couche étanche n'étant interrompue qu'à des endroits définis, la couche étanche étant conçue comme un joint élastique (34), le joint (34) étant réalisé de manière à adhérer à la surface extérieure de la deuxième chambre de mesure de gaz (3), de sorte que lors d'un remplacement de la deuxième chambre de mesure de gaz (3), le joint (34) est également remplacé.

2. Dispositif selon la revendication 1, **caractérisé en ce que** le capteur de gaz disposé dans la première chambre de mesure de gaz (2) dans la zone de la deuxième ouverture (24) comprend un émetteur (37) et un détecteur (38), qui sont situés sur des surfaces opposées intérieures de la première chambre de mesure de gaz (2), pour mesurer la concentration en gaz de dioxyde de carbone et/ou de monoxyde de carbone au moyen d'un procédé NDIR, c'est-à-dire un procédé à infrarouge non dispersif.

3. Dispositif selon l'une des revendications 1 à 2, **caractérisé en ce que** l'ouverture obturable comprend le dispositif de pompage de la chambre de mesure de (33) mentionné en tant qu'alternative, ledit dispositif de pompage de la chambre de mesure de (33) servant d'une ouverture ouverte entre la première chambre de mesure de gaz (2) et la deuxième chambre de mesure de gaz (3) en pompant l'air respirable de la première chambre de mesure de gaz (2) dans la deuxième chambre de mesure de gaz (3).

4. Dispositif selon l'une des revendications 1 à 3, **caractérisé en ce que** la deuxième chambre de mesure de gaz (3) comprend en outre une chambre partielle de mesure de gaz (35), dans ladite chambre partielle de mesure de gaz (35) étant disposé un catalyseur et/ou un oxydant et/ou un réactif pour l'oxydation du monoxyde d'azote en dioxyde d'azote.

5. Dispositif selon l'une des revendications 1 à 4, **caractérisé en ce que** la deuxième chambre de mesure de gaz (3) comprend au moins une zone partielle dans laquelle sont disposés des éléments de commande.

6. Dispositif selon la revendication 5, **caractérisé en ce que** la deuxième chambre de mesure de gaz (3) est réalisé de manière amovible par rapport à la première chambre de mesure de gaz (2) en desserrant une liaison mécanique, comme par exemple une liaison par encliquetage.

7. Dispositif selon l'une des revendications 1 à 6, **caractérisé en ce que** l'axe longitudinal du tube de l'embout buccal (1) et la première chambre de mesure de gaz (2) sont alignés selon un axe d'extension (12).

8. Dispositif selon l'une des revendications 1 à 7, **caractérisé en ce que**, un autre capteur de gaz est disposé dans l'embout buccal (1).

9. Dispositif selon l'une des revendications 1 à 8, **caractérisé en ce que**, le capteur de gaz dans la deuxième chambre de mesure de gaz (3) est réalisé comme un capteur à résistance ou un capteur à semi-conducteur et présente un corps de support avec une surface de corps de support, sur laquelle surface de corps de support sont disposées des pistes conductrices avec une surface de piste conductrice, sur laquelle est disposé un corps de mesure comprenant des tétracosanes et un liant comprenant un copolymère acrylique, un polymère de polyuréthane.

10. Dispositif selon l'une des revendications 1 à 9, **caractérisé en ce que** la pression dans la première chambre de mesure de gaz (2) et/ou le débit dans la première chambre de mesure de gaz (2) sont déterminés au moyen d'un premier capteur de pression (36) ou d'un capteur de débit, ledit capteur de pression (36) ou capteur de débit étant disposé en aval de la première ouverture (21), vu dans la direction de la deuxième voie d'écoulement (23).

11. Dispositif selon l'une des revendications 1 à 10, **caractérisé en ce que** le dispositif comprend un dispositif de pompage (6), ledit dispositif de pompage (6) étant en communication fluidique avec la première chambre de mesure de gaz (2) pour introduire un volume d'impulsion d'air dans la première chambre de mesure de gaz (2).

12. Dispositif selon l'une des revendications 1 à 11, **caractérisé en ce que** l'embout buccal (1) comprend un filtre antivirus et/ou un déshumidificateur.

13. Dispositif selon l'une des revendications 1 à 12, **caractérisé en ce que** le dispositif comprend un premier capteur de température disposé dans la première chambre de mesure de gaz (2) et un deuxième capteur de température disposé dans la deuxième chambre de mesure de gaz (3).

14. Dispositif selon l'une des revendications 1 à 13, **caractérisé en ce que** le dispositif comprend un premier capteur d'humidité disposé dans la première chambre de mesure de gaz (2) et un deuxième capteur d'humidité disposé dans la deuxième chambre de mesure de gaz (3).

15. Dispositif selon l'une des revendications 1 à 14, **caractérisé en ce que** le dispositif comprend :
- un capteur de pression pour déterminer la pression atmosphérique ambiante de l'environnement du dispositif et/ou
- un capteur de gaz pour déterminer la composition du gaz ambiant dans l'environnement du dispositif et/ou
- un capteur de température pour déterminer la température ambiante de l'environnement du dispositif et/ou
- un capteur d'humidité pour déterminer l'humidité ambiante de l'environnement du dispositif.

16. Dispositif selon l'une des revendications 1 à 15, **caractérisé en ce que** un dispositif de chauffage et/ou un dispositif de refroidissement pour chauffer ou refroidir l'air introduit dans la chambre de mesure de gaz (2, 3) et/ou les capteurs est disposé dans une entrée de chambre de gaz (5).

17. Dispositif selon l'une des revendications 1 à 16, **caractérisé en ce que** le dispositif comprend un moyen de communication de données pour transmettre les données déterminées au moyen du capteur à un appareil de traitement de données.

18. Dispositif selon l'une des revendications 1 à 17, **caractérisé en ce que** le dispositif comprend un compteur qui compte le nombre de mesures, la deuxième chambre de mesure de gaz (3) devant être remplacée lors d'un nombre défini de mesures.
